# EUROPEAN PATENT APPLICATION

(11) **EP 1 674 464 A1**
(43) Date of publication of application: **28.06.2006**
(21) Application number: 04030722.5
(22) Date of filing: 23.12.2004
(51) Int. Cl.: C07D 401/14, C07D 401/12, C07D 239/42, A61K 31/506, A61P 7/02

(54) **Novel thrombin inhibitors**

(71) Applicant: Santhera Pharmaceuticals (Schweiz) GmbH, 4410 Liestal (CH)
(72) Inventor: Bulat, Stephan., 69118 Heidelberg-Ziegelhausen (DE); Bosio, Sara, 64653 Lorsch (DE); Feurer, Achim, 69259 Wilhelmsfeld (DE); Papadopoulos, Michael Arthur, 69124 Heidelberg (DE); Rosenbaum, Claudia, 50354 Hürth (DE); Matassa, Victor Giulo, Barcelona 08198 (ES)
(74) Representative: Grünecker, Kinkeldey, Stockmair & Schwanhäusser Anwaltssozietät

(57) **Abstract**

The invention relates to compounds of formula (I) 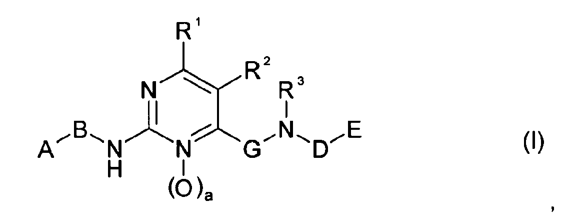 wherein A, B, R¹, R², G, R³, D and E have the meaning as cited in the description and the claims. Said compounds are useful as coagulants. The invention also relates to the production and use thereof as medicament.

## Description

The present invention relates to pyridines having an antithrombotic effect and their prodrugs useful as anticoagulants for the treatment or prophylaxis of thrombin related diseases.

Venous and arterial thromboembolism may cause pulmonary embolism, myocardial infarction and ischaemic stroke and hence are a major cause for morbidity and mortality. Therefore, significant efforts have been made to find effective antithrombotic therapies. The list of established drugs for the prevention of thrombus formation and embolisation include low molecular weight heparins, hirudin and its derivatives, aspirin, thienopyridine-type ADP receptor antagonists and glycoprotein lib/Illa receptor antagonists, as well as vitamin K antagonists. Several limitations caused some these therapies being of only limited use or leading to severe implications. These treatments have limited use because of severe side effects. These limitations in current therapies have stimulated the search for new and more efficient anticoagulants.

Thrombin is a serine protease present in blood plasma in the form of its precursor, prothrombin (Mann, K.G., *Biochemistry and physiology of blood coagulation, Thromb. Haemost.* 1999, 82, 165-74) and plays a central role in the mechanism of blood coagulation by converting the soluble plasma protein fibrinogen into the insoluble fibrin which forms a clot. In addition, thrombin transforms coagulation factor XII to factor Villa which covalently cross-links the fibrin strands. Thrombin is responsible for a variety of cellular actions mediated by binding to specific protease-activated receptors (O'Brien, P.J. et al. *Protease activated receptors: theme and variations. Oncogene* 2001, 20, 1570-81). In addition, thrombin is one of the most potent stimulators of platelet aggregation and also a potent mitogen for vascular muscle cells.

Due to its multiple physiological actions in the context of blood coagulation, thrombin is considered to be an extremely suitable target for the discovery and development of anticoagulative therapeutic agents.

Among the non-peptidic and peptidic inhibitors of enzymes of the coagulation cascade reported in the literature so far only a limited number are based on a pyrimidine core structure. In the US patent application US 2003/0158218 A1 unsubstituted and 5-cyano-substituted aminopyrimidine acetamides are reported to be useful in inhibiting thrombin and associated thrombotic occlusions. US patent 6,624,180 B2 describes 5-aryl-substituted aminopyrimidine acetamides which selectively inhibit certain proteases of the coagulation cascade.

In general, many of the so far reported thrombin inhibitors suffer from poor physicochemical properties (such as solubility) and/or poor ADME profile. As a consequence, there is still only one thrombin inhibitor that has passed all clinical trials (Ximelagatran) which in turn is currently in discussion due to elevated liver enzyme levels observed in man. Hence, there is still a high medical need for effective and safe antithrombotic agents.

Thus, the object of the present invention is to provide novel and selective compounds which can overcome at least some of the draw backs of compounds considered state-of-the-art.

Accordingly, the present invention provides compounds of formula (I): 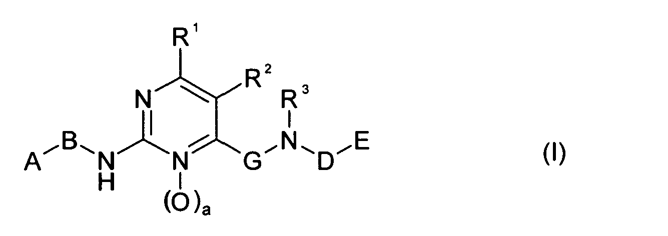 or a pharmaceutically acceptable salt thereof, wherein:
- a is: 0 or 1;
- R¹ is: hydrogen;
halogen;
CN;
CNO; or
C₁₋₄ alkyl, optionally substituted with one or more fluoro;
- R² is: hydrogen;
halogen;
CN;
CNO;
C₁₋₆ alkyl, optionally substituted with one or more fluoro;
C₃₋₆ cycloalkyl, optionally substituted with one or more fluoro; or
O-C₁₋₄ alkyl, optionally substituted with one or more fluoro;
- R³ is: hydrogen;
C₁₋₄ alkyl, optionally substituted with one or more fluoro; or
C₃₋₆ cycloalkyl, optionally substituted with one or more fluoro;
- A: is A¹, wherein A¹ is selected from the group consisting of:
phenyl;
naphthyl;
heterocycle containing 1, 2, 3 or 4 heteroatoms, which are the same or different and
selected from the group consisting of -O-, -S-, -S(O)-, -S(O₂)- -N=, -N(O)= and -N(R⁴)-; and
heterobicycle containing 1, 2, 3, 4, 5 or 6 heteroatoms, which are the same or different
and selected from the group consisting of -O-, -S-, -S(O)-, -S(O₂)-, -N=, -N(O)= and -N(R⁴)-; wherein A¹ is optionally substituted with one or independently from each other more of
A²;
A³;
halogen;
CN;
CNO;
-N(R⁵R⁶);
-OH;
=O, where the ring is at least partially saturated;
C₃₋₆ cycloalkyl;
-COOR⁷; or
-CONR⁸R⁹;
-S(O)₂NR^{8a}R^{9a}
and wherein R⁴, R⁵, R⁶ are independently selected from the group consisting of R^{7a}, -C(O)-R^{7a}, -C(O)O-R^{7a}, -C(O)NR^{7a}R^{7b}, -S(O)₂NR^{7a}R^{7b}, and S(O)₂-R^{7a}; and wherein R⁷, R^{7a}, R^{7b}, R⁸, R^{8a}, R⁹, R^{9a} are independently hydrogen or C₁₋₄ alkyl, wherein each C₁₄ alkyl is optionally substituted with one or more substituents independently selected from the group consisting of -COOH; -COO-C₁₋₄ alkyl; -OH; -NH₂; -NH-C₁₋₄ alkyl; -N(C₁₋₄ alkyl)₂; and C₃₋₆ cycloalkyl;

Optionally R⁴ is a bond to directly attach A to B;
- A² is: selected from the group consisting of A⁴, -O-A⁴ and -N(R¹⁰)-A⁴, wherein A⁴ is phenyl or a heterocycle containing 1, 2, 3 or 4 heteroatoms, which are the same or different and selected from the group consisting of -O-, -S-, -S(O)-, -S(O₂)-, -N=, -N(O)= and -N(R¹¹)-; wherein A⁴ is optionally substituted with one or independently from each other more of
fluoro;
chloro;
-N(R¹²R¹³)
C₁₋₄ alkyl or -O-C₁₋₄ alkyl, both optionally substituted with one or
independently from each other more of fluoro or -N(R¹⁴R¹⁵);
and wherein R¹⁰, R¹², R¹³, R¹⁴, R¹⁵ are independently hydrogen or C₁₋₄ alkyl;
and wherein R¹¹ is selected from the group consisting of hydrogen, C₁₋₄ alkyl
and -C(O)-C₁₋₄ alkyl;
- A³: is selected from the group consisting of C₁₋₆ alkyl, -O-C₁₋₆ alkyl and -N(R¹⁶)-C₁₋₆ alkyl, wherein the C₁₋₆ alkyl group is optionally substituted with one or independently from each other more of
fluoro;
-N(R¹⁷R¹⁸);
A⁵;
and/or A³ is optionally interrupted with one or more oxygen; and wherein R¹⁶, R¹⁷, R¹⁸ are independently hydrogen or C₁₋₄ alkyl;
- A⁵: is phenyl or a heterocycle containing 1, 2, 3 or 4 heteroatoms, which are the same or different and selected from the group consisting of -O-, -S-, -S(O)-, -S(O₂)-, -N=, - N(O)= and -N(R¹⁹)-; wherein A⁵ is optionally substituted with one or independently from each other more of
fluoro;
chloro;
-N(R²⁰R²¹)
C₁₋₄ alkyl or -O-C₁₋₄ alkyl, both optionally substituted with one or independently from each other more of fluoro or -N(R²²R²³);
and wherein R¹⁹ is selected from the group consisting of hydrogen, C₁₋₄ alkyl and -C(O)-C₁₋₄ alkyl;
and wherein R²⁰, R²¹, R²², R²³ are independently hydrogen or C₁₋₄ alkyl;
- B is: selected from the group consisting of -Y-Z-; -Y-Z-C(O)-; -Y-Z-O-C(O)-; -Y-Z-S(O)₂-; and -Y-Z-NH-C(O)- wherein
Y is a bond, -O-, -S-, -N(R²⁴)-, -N(R²⁵)-C(O)-, -C(O)-N(R²⁶)-, or -C(O)-;
Z is C₁₋₆ alkyl,
optionally interrupted with oxygen, sulfur or -N(R²⁷)-
and/or optionally substituted with one or independently from each
other more of
halogen;
CN;
CNO;
C₃₋₆ cycloalkyl;
-COOR²⁸;
-CON(R²⁹R³⁰)
and/or optionally one chain carbon forms part of a C₃₋₆ cycloalkyl;
and wherein R²⁴, R²⁵, R²⁶, R²⁷, R²⁸, R²⁹, R³⁰ are independently
hydrogen; or
C₁₋₄ alkyl, optionally substituted with -COOR³¹ or -CON(R³²R³³)
wherein R³¹, R³², R³³ are independently hydrogen or
C₁₋₄ alkyl;
- G is: -CH(R³⁷)-C(R³⁸R³⁹)-;
-CH(R³⁷)-C(R⁴⁰R⁴¹)-C(R³⁸R³⁹)-;
wherein R³⁷, R⁴⁰, R⁴¹ are independently
hydrogen;
F;
C₁₋₄ alkyl, optionally substituted with one or more fluoro;
C₃₋₆ cycloalkyl, optionally substituted with one or more fluoro;
wherein R³⁸, R³⁹ are independently
hydrogen;
C₁₋₄ alkyl, optionally substituted with one or more fluoro;
C₃₋₆ cycloalkyl, optionally substituted with one or more fluoro; or R³⁸ and R³⁹, or R⁴⁰ and R⁴¹ or R³⁸ and R⁴⁰ form together C₃₋₆ cycloalkyl, optionally
substituted with one or more fluoro, -OH, C₁₋₄ alkyl; or R³⁷ and R³⁸ in the case of -CH(R³⁷)-C(R³⁸R³⁹)-, or R³⁷ and R⁴⁰ in the case of -CH(R³⁷)-C(R⁴⁰R⁴¹)-C(R³⁸R³⁹)-, form together C₃₋₆ cycloalkyl, optionally
substituted with one or more fluoro, -OH, C₁₋₄ alkyl;
- D is: C₁₋₆ alkyl,
optionally interrupted with oxygen, sulfur or -N(R⁴²)-
and/or optionally substituted with halogen, CN; CNO; C₃₋₆ cycloalkyl;
and/or optionally one chain carbon or two vicinal carbons form part of a C₃₋₆ cycloalkyl, wherein R⁴² is selected from the group consisting of hydrogen, C₁₋₄ alkyl, C₃₋₆ cycloalkyl and -C(O)-C₁₋₄ alkyl;
- E is: E¹, wherein E¹ is selected from the group consisting of
phenyl;
naphthyl;
heterocycle containing 1, 2, 3 or 4 heteroatoms, which are the same or different and
selected from the group consisting of -O-, -S-, -S(O)-, -S(O₂)-, -N=, -N(O)= and -N(R⁴³)-; and heterobicycle containing 1, 2, 3, 4, 5 or 6 heteroatoms, which are the same or different
and selected from the group consisting of -O-, -S-, -S(O)-, -S(O₂)-, -N=, -N(O)= and -N(R⁴⁴)-;
wherein E¹ is optionally substituted with one or independently from each other more of
E²;
E³;
halogen;
CN;
CNO;
-N(R⁴⁵R⁴⁶);
-OH;
=O, where the ring is at least partially saturated;
C₃₋₆ cycloalkyl;
-COOR⁴⁷; or
-CONR⁴⁸R⁴⁹;
-S(O)₂NR^{48a}R^{49a};
and wherein R⁴³, R⁴⁴,R⁴⁵, R⁴⁶ R^{48a}, R^{49a} are independently selected from the
group consisting of
hydrogen;
C₁₋₄ alkyl optionally substituted with -OH and/or F;
and -C(O)-C₁₋₄ alkyl optionally substituted with -OH and/or F;
and wherein R⁴⁷, R⁴⁸, R⁴⁹ are independently hydrogen or C₁₋₄ alkyl, optionally substituted with -OH and/or F;
- E² is: selected from the group consisting of E⁴, -C(O)-E⁴, -O-E⁴ and -N(R⁵⁰)-E⁴,
wherein E⁴ is phenyl or heterocycle containing 1, 2, 3 or 4 heteroatoms, which are the same or different and selected from the group consisting of -O-, -S-, -S(O)-, -S(O₂)- -N=, -N(O)= and -N(R⁵¹)-; wherein E⁴ is optionally substituted with one or independently from each other more of
fluoro;
chloro;
cyano;
=O, where the ring is at least partially saturated;
-N(R⁵²R⁵³);
C₁₋₄ alkyl; or
-O-C₁₋₄ alkyl;
and wherein R⁵⁰, R⁵², R⁵³ are independently hydrogen or C₁₋₄ alkyl, optionally substituted with -OH;
and wherein R⁵¹ is selected from the group consisting of
hydrogen;
C₁₋₄ alkyl, optionally substituted with -OH; and
-C(O)-C₁₋₄ alkyl, optionally substituted with -OH;
- E³: is selected from the group consisting of C₁₋₆ alkyl, -O-C₁₋₆ alkyl; -N(R⁵⁴)-C₁₋₆ alkyl, wherein E³ is optionally substituted with one or independently from each other more of
fluoro;
OH;
-N(R⁵⁵R⁵⁶);
E⁵;
and/or E³ is optionally interrupted with one or more oxygen; and wherein R⁵⁴, R⁵⁵, R⁵⁶ are independently hydrogen or C₁₋₄alkyl, optionally substituted with -OH;
- E⁵: is phenyl or heterocycle containing 1, 2, 3 or 4 heteroatoms, which are the same or different and selected from the group consisting of -O-, -S-, -S(O)-, -S(O₂)-, -N=, -N(O)= and -N(R⁵⁷)-; wherein E⁵ is optionally substituted with one or independently from each other more of
fluoro;
chloro;
cyano;
=O, where the ring is at least partially saturated;
-N(R⁵⁸R⁵⁹);
C₁₋₄ alkyl or
-O-C₁₋₄ alkyl;
and wherein R⁵⁷ is independently selected from the group consisting of hydrogen;
C₁₋₄ alkyl, optionally substituted with -OH; and
-C(O)-C₁₋₄ alkyl, optionally substituted with -OH;
and wherein R⁵⁸, R⁵⁹ are independently hydrogen or C₁₋₄ alkyl, optionally substituted with -OH.

Within the meaning of the present invention the terms are used as follows:
"Alkyl" means a straight-chain or branched carbon chain that may contain double or triple bonds.
"C₁₋₄ Alkyl" means an alkyl chain having 1 - 4 carbon atoms, e.g. at the end of a molecule methyl, ethyl, -CH=CH₂, -C≡CH, n-propyl, isopropyl, -CH=CH-CH₃, -CH₂₋CH=CH₂, n-butyl, isobutyl, -CH=CH-CH₂-CH₃, -CH=CH-CH=CH₂, sec-butyl tert-butyl or amidst, e.g. -CH₂-, -CH₂-CH₂-, -CH=CH-, -CH(CH₃)-, -C(CH₂)-, -CH₂-CH₂-CH₂-, -CH(C₂H₅)-, -CH(CH₃)₂-.
"C₁₋₆ Alkyl" means an alkyl chain having 1 - 6 carbon atoms, e.g. C₁₋₄ Alkyl, methyl, ethyl, -CH=CH₂, -C≡CH, n-propyl, isopropyl, -CH=CH-CH₃, -CH₂-CH=CH₂, n-butyl, isobutyl, -CH=CH-CH₂-CH₃, -CH=CH-CH=CH₂, sec-butyl tert-butyl, n-pentane, n-hexane, or amidst, e.g. -CH₂-, -CH₂-CH₂-, -CH=CH-, -CH(CH₃)-, -C(CH₂)-, -CH₂-CH₂₋CH₂-, -CH(C₂H₅)-, -CH(CH₃)₂-.

An alkyl chain "interrupted" with a heteroatom means that between two carbon atoms or at the end of the alkyl chain a heteroatom, e.g. nitrogen, oxygen or sulfur, is added. This includes for example C₁₋₄ alkyl interrupted by an oxygen atom, e.g. -CH₂-OH, -CH₂-O-CH₃, CH₂-CH₂-OH, -C₃H₆-OCH₃.

Each hydrogen of a carbon or heteroatom of the alkyl chain or interrupted alkyl chain may be replaced by a substituent.

"C₃₋₆ Cycloalkyl" means a cyclic alkyl chain having 3 - 6 carbon atoms, e.g. cyclopropyl, cyclobutyl, cyclopentyl, cyclohexyl, cyclohexenyl. Each hydrogen of a cycloalkyl carbon may be replaced by a substituent.

"Halogen" means fluoro, chloro, bromo and so called pseudo-halogens, i.e. -CN or -CNO.

"Heterocycle" means a cyclopentane, cyclohexane or cycloheptane ring that may contain up to the maximum number of double bonds (aromatic or non-aromatic ring which is fully, partially or un-saturated) wherein at least one carbon atom up to a maximum number of carbon atoms, as indicated, is replaced by a heteroatom ("containing" or "having" a heteroatom) and wherein the ring is linked to the rest of the molecule via a carbon or nitrogen atom. Examples for a heterocycle are furan, thiophene, pyrrole, pyrroline, imidazole, imidazoline, pyrazole, pyrazoline, oxazole, oxazoline, isoxazole, isoxazoline, thiazole, thiazoline, isothiazole, isothiazoline, thiadiazole, thiadiazoline, tetrahydrofuran, tetrahydrothiophene, pyrrolidine, imidazolidine, pyrazolidine, oxazolidine, isoxazolidine, thiazolidine, isothiazolidine, thiadiazolidine, sulfolane, pyran, dihydropyran, tetrahydropyran, imidazolidine, pyridine, pyridazine, pyrazine, pyrimidine, piperazine, piperidine, morpholine, tetrazole, triazole, triazolidine, tetrazolidine, azepine or homopiperazine.

"Heterobicycle" means a heterocycle which is condensed with phenyl or an additional heterocycle to form a bicyclic ring system. "Condensed" to form a bicyclic ring means that two rings are attached to each other by sharing two ring atoms. Examples for a heterobicycle are indole, indoline, benzofuran, benzothiophene, benzoxazole, benzisoxazole, benzothiazole, benzisothiazole, benzimidazole, benzimidazoline, quinoline, quinazoline, dihydroquinazoline, dihydroquinoline, isoquinoline, tetrahydroisoquinoline, dihydroisoquinoline, benzazepine, purine or pteridine.

When a divalent group is contemplated, i.e., the moiety B, G or D, then this group is defined in such a manner that it is bonded to the connecting points of the molecule in exactly the same order as shown herein. For example, when the moiety D represents -CH₂-CH₂-NH-, then the CH₂ terminus is bonded to the N of the amine moiety according to formula (I), and the NH terminus is bonded to the E moiety of the structure according to formula (I).

Preferred compounds of the formula (I) are those compounds in which one or more of the residues contained therein have the meanings given below, with all combinations of preferred substituent definitions being a subject of the present invention. With respect to all preferred compounds of the formula (I) the present invention also includes all tautomeric and stereoisomeric forms and mixtures thereof in all ratios, and their pharmaceutically acceptable salts.

In preferred embodiments of the present invention, the substituents R¹ - R³, A, B, X, G, D and E of the formula (I) independently from each other have the following meaning. Hence, one or more of the substituents R¹ - R³, A, B, X, G, D and E can have the preferred or more preferred meanings given below.

R¹ is preferably hydrogen.

R² is preferably hydrogen, chloro, -CH₃, -CH₂-CH₃, -CH₂-CH₂-CH₃, -CH₂-CH₂-CH₂-CH₃, -CH₂F, -CHF₂ or -CN.

R³ is preferably hydrogen.

Preferably in A is A¹ phenyl or heterocycle containing up to 1, 2, 3 or 4 heteroatoms, which are the same or different and selected from the group consisting of -O-, -S-, - S(O)-, -S(O₂)-, -N=, -N(O)= and -N(R⁴)-, wherein R⁴ has the meaning as indicated above.

More preferred A¹ is selected from the group consisting of phenyl, pyridine, pyridine-N oxide, piperidine, morpholine, and pyrrolidine.

Preferably, R⁴ is a bond, hydrogen, -COOC₁₋₄ alkyl, methyl, ethyl, 2-hydroxyethyl, -COOH, -CH₂-COOH, -CH₂-COO-C₁₋₄ alkyl or cyclopropylmethyl and preferably, A¹ is optionally substituted with up to 1, 2, 3 or 4 F. When R⁴ is a bond this means that the nitrogen of the heterocycle represented by A¹ is directly bonded to the group B.

Preferably, B is -Y-Z-.

Preferably in B is Y preferably a bond or -O- and Z is -C(R⁶⁰R⁶¹)- or -C(R⁶⁰R⁶¹)-C(R⁶²R⁶³)-, wherein
R⁶⁰, R⁶¹, R⁶², R⁶³ are independently hydrogen, -C(O)NH₂, -COOH, -CH₂-COOH,
-CH₂-C(O)NH₂, fluoro, methyl, cyclopropyl or
R⁶⁰ and R⁶¹ form a cyclopropyl ring or
R⁶² and R⁶³ form a cyclopropyl ring or
R⁶⁰ and R⁶² form a cyclopropyl or cyclobutyl ring.

Preferably R⁶⁰, R⁶¹, R⁶², R⁶³ are independently hydrogen, fluoro or -C(O)NH₂-.

G is preferably G is -CH(R⁶⁴)-C(R⁶⁵R⁶⁶)-; wherein R⁶⁴ is hydrogen, F, methyl, -CH₂F, -CHF₂, CF₃ or cyclopropyl; and R⁶⁵, R⁶⁶ are independently hydrogen, methyl, -CH₂F, -CHF₂, CF₃ or cyclopropyl or R⁶⁵, R⁶⁶ form together cyclopropyl.

It is also preferred that G is -CH₂-CH₂-.

Preferably, D is -CH₂-, -CF₂-, -CH(CH₃)-, -C(CH₃)₂- or D¹-D², where D¹ and D² are independently -CH₂-, -CF₂-, -CH(CH₃)- or -C(CH₃)₂- and wherein D² is optionally -CH₂₋NH-. More preferred D is -CH₂-, -CH(CH₃)-, -CH₂-CH₂-, -CH₂-CF₂ or -CH₂-CH₂-NH-.

Preferably, E is selected from the group consisting of phenyl; heterocycle containing 1, 2 or 3 heteroatoms, which are the same or different and selected from the group consisting of -O-, -N=, -N(O)- and -NH-; and heterobicycle containing 1, 2 or 3 heteroatoms, which are the same or different and selected from the group consisting of -O-, -N=, and -NH-; and wherein E is optionally substituted with 1 or 2 substituents which are the same or different and selected from the group consisting of CN, F, Cl, C₁₋₄alkyl, OH, O-C₁₋₄ alkyl, NH₂, NH-C₁₋₄ alkyl, N(C₁₋₄ alkyl)₂, C(O)NH₂, C(O)NH-C₁₋₄ alkyl, and C(O)N(C₁₋₄ alkyl)₂, wherein each C₁₋₄ alkyl is optionally substituted with one or more substituents independently selected from OH and F. It is more preferred that E is phenyl, pyridine, benzimidazole, indazole, quinoline, isoquinoline, pyridine-(N)-oxide, benzothiophene, indole, azaindole, benzofuran, benzisoxazole, benzoxazole, benzothiazole.

It s also preferred that E is selected from the group consisting of 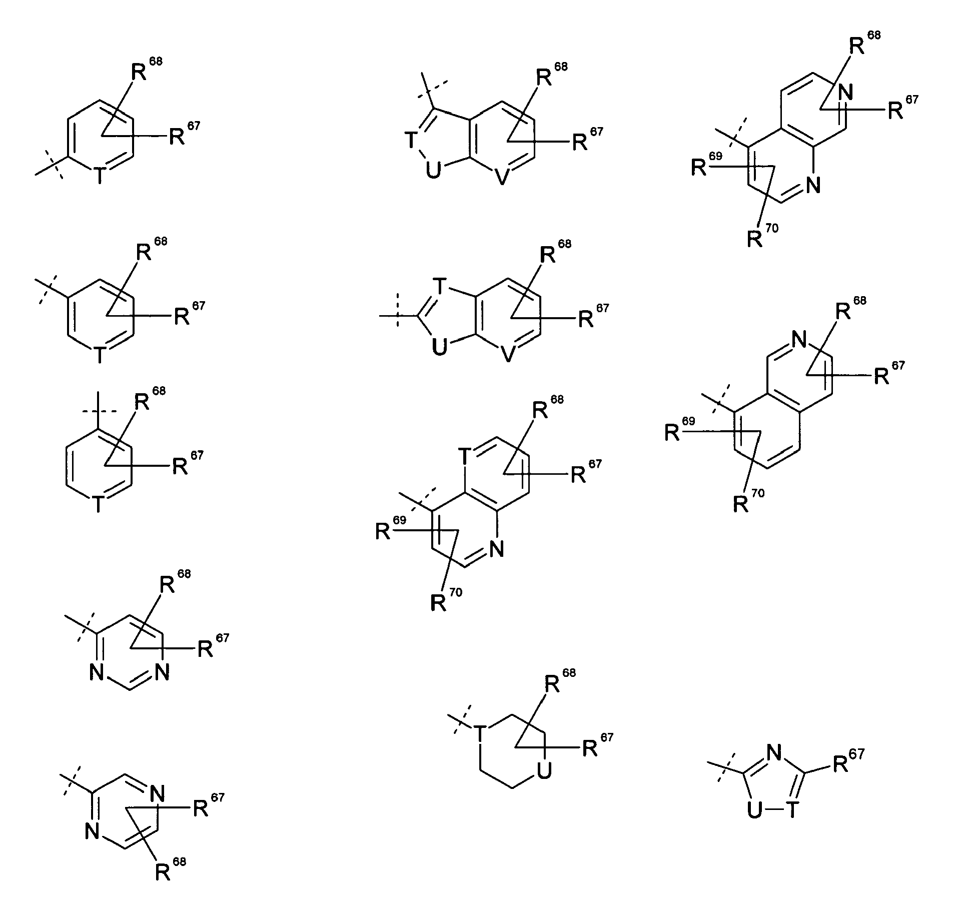 wherein
T and V are independently =CH-, =CR⁷¹, =N- or =N(O)-;
U is -NH-, -NR⁷²-, -O-, or -S-, wherein
R⁶⁷, R⁶⁸, R⁶⁹, R⁷⁰, R⁷¹ are independently selected from the group consisting of
hydrogen;
C₃₋₆ cycloalkyl;
E⁶;
E⁷;
halogen;
CN;
CNO;
-N(R⁷³R⁷⁴);
-OH; and
-COOR⁷⁵ or -C(O)NR⁷⁶R⁷⁷;
and wherein R⁷², R⁷³, R⁷⁴ are independently
hydrogen;
C₁₋₄ alkyl; or
-C(O)-C₁₋₄ alkyl;
and wherein R⁷⁵, R⁷⁶, R⁷⁷ are independently
hydrogen; or
C₁₋₄ alkyl;

E⁶ is selected from the group consisting of C₁₋₆ alkyl; -O-C₁₋₆ alkyl; and -N(R⁷⁸)-C₁₋₆ alkyl, wherein the C₁₋₆ alkyl group is optionally substituted with one or more of
F;
CN;
CNO;
-N(R⁷⁹R⁸⁰);
phenyl, optionally substituted with chloro;
heterocycle containing 1, 2, 3 or 4 heteroatoms, which are the same or different and selected from the group consisting of -O-, -S-, -S(O)-, -S(O₂)-, -N=,
-N(O)= and -N(R⁸¹)_, optionally substituted with chloro;
and/or E⁶ is optionally interrupted by one or more of oxygen;
and wherein R⁷⁸, R⁷⁹ , R⁸⁰, R⁸¹ are independently hydrogen, C₁₋₄alkyl;

E⁷ is selected from the group consisting of E⁸; -O-E⁸; -N(R ⁸² )-E⁸; and -C(O)-E⁸, wherein E⁸ is phenyl or heterocycle containing 1, 2, 3 or 4 heteroatoms, which are the same or different and selected from the group consisting of -O-, -S-, -S(O)-, -S(O₂)-, -N=, -N(O)= and -N(R⁸³)-; and wherein E⁸ is optionally substituted with chloro or -N(R⁸⁴R⁸⁵); and wherein R⁸², R⁸³, R⁸⁴, R⁸⁵ are independently hydrogen or C₁₋₄ alkyl.

Preferably, R⁶⁷, R⁶⁸, R⁶⁹, R⁷⁰, R⁷¹ are independently hydrogen, fluoro, chloro, cyano, phenyl, chlorophenyl, methyl, methoxy, amino, monomethyl amino, dimethyl amino, pyrrolyl, diazolyl, triazolyl, and tetrazolyl.

Compounds of the formula (I) in which some or all of the above-mentioned groups have the preferred or more preferred meanings are also an object of the present invention.

A preferred embodiment of compounds according to present invention is shown below:

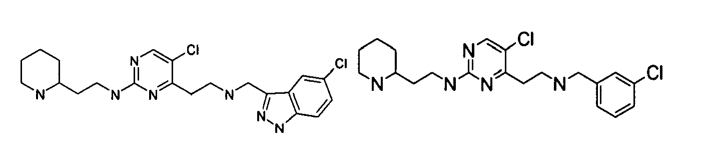

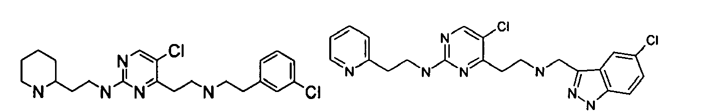

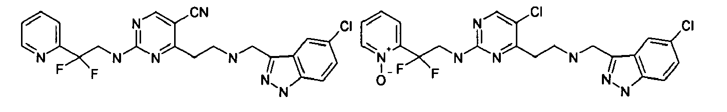

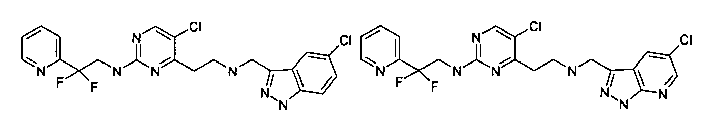

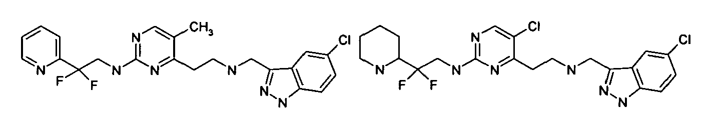

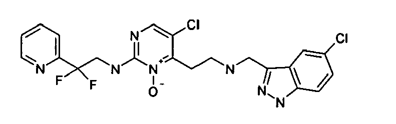

Furthermore, the present invention provides prodrugs of the compounds of the invention as described above.

"Prodrug" means a derivative that is converted into a compound according to the present invention by a reaction with an enzyme, gastric acid or the like under a physiological condition in the living body, e.g. by oxidation, reduction, hydrolysis or the like, each of which is carried out enzymatically. Examples of the prodrug are compounds, wherein the amino group in a compound of the present invention is acylated, alkylated or phosphorylated to form, e.g., eicosanoylamino, alanylamino, pivaloyloxymethylamino or wherein the hydroxyl group is acylated, alkylated, phosphorylated or converted into the borate, e.g. acetyloxy, palmitoyloxy, pivaloyloxy, succinyloxy, fumaryloxy, alanyloxy or wherein the carboxyl group is esterified or amidated. These compounds can be produced from compounds of the present invention according to well-known methods.

Where tautomerism, like e.g. keto-enol tautomerism, of compounds of general formula (I) or their prodrugs may occur, the individual forms, like e.g. the keto and enol form, are claimed separately and together as mixtures. Same applies for stereoisomers, like e.g. enantiomers, cis/trans isomers, conformers and the like.

In case the compounds according to formula (I) contain one or more acidic or basic groups, the invention also comprises their corresponding pharmaceutically or toxicologically acceptable salts, in particular their pharmaceutically utilizable salts. Thus, the compounds of the formula (I) which contain acidic groups can be present on these groups and can be used according to the invention, for example, as alkali metal salts, alkaline earth metal salts or as ammonium salts. More precise examples of such salts include sodium salts, potassium salts, calcium salts, magnesium salts or salts with ammonia or organic amines such as, for example, ethylamine, ethanolamine, triethanolamine or amino acids. Compounds of the formula (I) which contain one or more basic groups, i.e. groups which can be protonated, can be present and can be used according to the invention in the form of their addition salts with inorganic or organic acids. Examples for suitable acids include hydrogen chloride, hydrogen bromide, phosphoric acid, sulfuric acid, nitric acid, methanesulfonic acid, p-toluenesulfonic acid, naphthalenedisulfonic acids, oxalic acid, acetic acid, tartaric acid, lactic acid, salicylic acid, benzoic acid, formic acid, propionic acid, pivalic acid, diethylacetic acid, malonic acid, succinic acid, pimelic acid, fumaric acid, maleic acid, malic acid, sulfaminic acid, phenylpropionic acid, gluconic acid, ascorbic acid, isonicotinic acid, citric acid, adipic acid, and other acids known to the person skilled in the art. If the compounds of the formula (I) simultaneously contain acidic and basic groups in the molecule, the invention also includes, in addition to the salt forms mentioned, inner salts or betaines (zwitterions). The respective salts according to the formula (I) can be obtained by customary methods which are known to the person skilled in the art like, for example by contacting these with an organic or inorganic acid or base in a solvent or dispersant, or by anion exchange or cation exchange with other salts. The present invention also includes all salts of the compounds of the formula (I) which, owing to low physiological compatibility, are not directly suitable for use in pharmaceuticals but which can be used, for example, as intermediates for chemical reactions or for the preparation of pharmaceutically acceptable salts.

The present invention provides compounds of general formula (I) or their prodrugs as anticoagulants or thrombin inhibitors. This includes compounds for inhibiting thrombus formation, and inhibiting embolus formation in a mammal, inhibiting loss of blood platelets, inhibiting formation of blood platelet aggregates, inhibiting formation of fibrin. These compounds may optionally include anticoagulants, antiplatelet agents, and thrombolytic agents. The compounds can be added to blood, blood products, or mammalian organs in order to effect the desired inhibitions.

Furthermore, the invention includes compounds of formula (I) or their prodrugs or pharmaceutically acceptable salts for use as a medicament and their use for the manufacture of a medicament for the treatment or prophylaxis of thromboembolism, thrombosis, artherosclerosis, unstable angina, refractory angina, myocardial infarction, transient ischemic attacks, atrial fibrillation, thrombotic stroke, embolic stroke, deep vein thrombosis, disseminated intravascular coagulation, ocular build up of fibrin, and reocclusion or restenosis of recanalized vessels.

The present invention also includes pharmaceutical compositions comprising a compound of formula (I) or their prodrugs or a mixture of compounds or prodrugs or a pharmaceutically acceptable salt thereof together with a pharmaceutically acceptable carrier. Optionally, these pharmaceutical compositions may additionally comprise one or more known anticoagulants.

The therapeutic use and method of using anticoagulants or thrombin inhibitors like the compounds of formula (I) of the present invention or their prodrugs or their use for the manufacture of a medicament are well known in the art and are described in more detail in US 2003/01582218 A1 which is herewith incorporated by reference.

Accordingly, therapies based on anticoagulants are indicated for the prevention and treatment of a variety of thrombotic conditions, particularly coronary artery and cerebrovascular disease. Those experienced in this field are readily aware of the circumstances requiring anticoagulant therapy. The term "patient" used herein is taken to mean mammals such as primates, including humans, sheep, horses, cattle, pigs, rabbits, dogs, cats, rats, and mice.

Compounds of the present invention are useful for treating or preventing venous thromboembolism (e. g. obstruction or occlusion of a vein by a detached thrombus; obstruction or occlusion of a lung artery by a detached thrombus), cardiogenic thromboembolism (e. g. obstruction or occlusion of the heart by a detached thrombus), arterial thrombosis (e. g. formation of a thrombus within an artery that may cause infarction of tissue supplied by the artery), atherosclerosis (e. g. arteriosclerosis characterized by irregularly distributed lipid deposits) in mammals, and for lowering the propensity of devices that come into contact with blood to clot blood.

Examples of venous thromboembolism which may be treated or prevented with compounds of the invention include obstruction of a vein, obstruction of a lung artery (pulmonary embolism), deep vein thrombosis, thrombosis associated with cancer and cancer chemotherapy, thrombosis inherited with thrombophilic diseases such as Protein C deficiency, Protein S deficiency, antithrombin III deficiency, and Factor V Leiden, and thrombosis resulting from acquired thrombophilic disorders such as systemic lupus erythematosus (inflammatory connective tissue disease). Also with regard to venous thromboembolism, compounds of the invention are useful for maintaining patency of indwelling catheters.

Examples of cardiogenic thromboembolism which may be treated or prevented with compounds of the invention include thromboembolic stroke (detached thrombus causing neurological affliction related to impaired cerebral blood supply), cardiogenic thromboembolism associated with atrial fibrillation (rapid, irregular twitching of upper heart chamber muscular fibrils), cardiogenic thromboembolism associated with prosthetic heart valves such as mechanical heart valves, and cardiogenic thromboembolism associated with heart disease.

Examples of arterial thrombosis include unstable angina (severe constrictive pain in chest of coronary origin), myocardial infarction (heart muscle cell death resulting from insufficient blood supply), ischemic heart disease (local anemia due to obstruction (such as by arterial narrowing) of blood supply), reocclusion during or after percutaneous transluminal coronary angioplasty, restenosis after percutaneous transluminal coronary angioplasty, occlusion of coronary artery bypass grafts, and occlusive cerebrovascular disease. Also with regard to arterial thrombosis, compounds of the present invention are useful for maintaining patency in arteriovenous cannulas.

Examples of atherosclerosis include arteriosclerosis.

Thrombin inhibition is useful not only in the anticoagulant therapy of individuals having thrombotic conditions, but is useful whenever inhibition of blood coagulation is required such as to prevent coagulation of stored whole blood and to prevent coagulation in other biological samples for testing or storage. Thus, the thrombin inhibitors can be added to or contacted with any medium containing or suspected of containing thrombin and in which it is desired that blood coagulation be inhibited, e. g., when contacting the mammal's blood with material selected from the group consisting of vascular grafts, stents, orthopedic prosthesis, cardiac prosthesis, and extracorporeal circulation systems.

Examples of devices that come into contact with blood include vascular grafts, stents, orthopedic prosthesis, cardiac prosthesis, and extracorporeal circulation systems The thrombin inhibitors of the invention can be administered in such oral forms as tablets, capsules (each of which includes sustained release or timed release formulations), pills, powders, granules, elixirs, tinctures, suspensions, syrups, and emulsions. Likewise, they may be administered in intravenous (bolus or infusion), intraperitoneal, subcutaneous, or intramuscular form, all using forms well known to those of ordinary skill in the pharmaceutical arts. An effective but nontoxic amount of the compound desired can be employed as an anti-aggregation agent.

For treating ocular build up of fibrin, the compounds may be administered intraocularly or topically as well as orally or parenterally.

The compounds of the present invention can be administered in the form of a depot injection or implant preparation which may be formulated in such a manner as to permit a sustained release of the active ingredient. The active ingredient can be compressed into pellets or small cylinders and implanted subcutaneously or intramuscularly as depot injections or implants. Implants may employ inert materials such as biodegradable polymers or synthetic silicones, for example, Silastic, silicone rubber or other polymers manufactured by the Dow-Corning Corporation.

The compounds of the present invention can also be administered in the form of liposome delivery systems, such as small unilamellar vesicles, large unilamellar vesicles and multilamellar vesicles. Liposomes can be formed from a variety of phospholipids, such as cholesterol, stearylamine or phosphatidylcholines.

The compounds of the present invention may also be delivered by the use of monoclonal antibodies as individual carriers to which the compound molecules are coupled. The compounds of the present invention may also be coupled with soluble polymers as targetable drug carriers. Such polymers can include polyvinylpyrrolidone, pyran copolymer, polyhydroxy-propyl-methacrylamide-phenol, polyhydroxyethylaspartamide-phenol, or polyethyleneoxide-polylysine substituted with palmitoyl residues. Furthermore, the thrombin inhibitors may be coupled to a class of biodegradable polymers useful in achieving controlled release of a drug, for example, polylactic acid, polyglycolic acid, copolymers of polylactic and polyglycolic acid, polyepsilon caprolactone, polyhydroxy butyric acid, polyorthoesters, polyacetals, polydihydropyrans, polycyanoacrylates and cross linked or amphipathic block copolymers of hydrogels.

The dosage regimen utilizing the thrombin inhibitors is selected in accordance with a variety of factors including type, species, age, weight, sex and medical condition of the patient; the severity of the condition to be treated; the route of administration; the renal and hepatic function of the patient; and the particular compound or salt thereof employed. An ordinarily skilled physician or veterinarian can readily determine and prescribe the effective amount of the drug required to prevent, counter, or arrest the progress of the condition.

Oral dosages of the compounds of the present invention, when used for the indicated effects, will range between about 0.01 mg per kg of body weight per day (mg/kg/day) to about 30 mg/kg/day, preferably 0.025-7.5 mg/kg/day, more preferably 0.1-2.5 mg/kg/day, and most preferably 0.1-0.5 mg/kg/day (unless specified otherwise, amounts of active ingredients are on free base basis). For example, an 80 kg patient would receive between about 0.8 mg/day and 2.4 g/day, preferably 2-600 mg/day, more preferably 8-200 mg/day, and most preferably 8-40 mg/kg/day. A suitably prepared medicament for once a day administration would thus contain between 0.8 mg and 2.4 g, preferably between 2 mg and 600 mg, more preferably between 8 mg and 200 mg, and most preferably 8 mg and 40 mg, e. g., 8 mg, 10 mg, 20 mg and 40 mg. Advantageously, the compounds of the present invention may be administered in divided doses of two, three, or four times daily. For administration twice a day, a suitably prepared medicament would contain between 0.4 mg and 4 g, preferably between 1 mg and 300 mg, more preferably between 4 mg and 100 mg, and most preferably 4 mg and 20 mg, e. g., 4 mg, 5 mg, 10 mg and 20 mg.

Intravenously, the patient would receive the active ingredient in quantities sufficient to deliver between 0.025-7.5 mg/kg/day, preferably 0.1-2.5 mg/kg/day, and more preferably 0.1-0.5 mg/kg/day. Such quantities may be administered in a number of suitable ways, e. g. large volumes of low concentrations of active ingredient during one extended period of time or several times a day, low volumes of high concentrations of active ingredient during a short period of time, e. g. once a day. Typically, a conventional intravenous formulation may be prepared which contains a concentration of active ingredient of between about 0.01-1.0 mg/ml, e. g. 0.1 mg/ml, 0.3 mg/ml, and 0.6 mg/ml, and administered in amounts per day of between 0.01 ml/kg patient weight and 10.0 ml/kg patient weight, e. g. 0.1 ml/kg, 0.2 ml/kg, 0.5 ml/kg. In one example, an 80 kg patient, receiving 8 ml twice a day of an intravenous formulation having a concentration of active ingredient of 0.5 mg/ml, receives 8 mg of active ingredient per day. Glucuronic acid, L-lactic acid, acetic acid, citric acid or any pharmaceutically acceptable acid/conjugate base with reasonable buffering capacity in the pH range acceptable for intravenous administration may be used as buffers. Consideration should be given to the solubility of the drug in choosing an appropriate buffer and pH of a formulation, depending on solubility of the drug to be administered, is readily made by a person having ordinary skill in the art.

The compounds of the present invention can also be administered in intranasal form via topical use of suitable intranasal vehicles, or via transdermal routes, using those forms of transdermal skin patches well known to those of ordinary skill in that art. To be administered in the form of a transdermal delivery system, the dosage administration will, or course, be continuous rather than intermittent throughout the dosage regime.

The compounds of the present invention are typically administered as active ingredients in admixture with suitable pharmaceutical diluents, excipients or carriers (collectively referred to herein as "carrier" materials) suitably selected with respect to the intended form of administration, that is, oral tablets, capsules, elixirs, syrups and the like, and consistent with convention pharmaceutical practices.

For instance, for oral administration in the form of a tablet or capsule, the active drug component can be combined with an oral, non-toxic, pharmaceutically acceptable, inert carrier such as lactose, starch, sucrose, glucose, methyl cellulose, magnesium stearate, dicalcium phosphate, calcium sulfate, mannitol, sorbitol and the like; for oral administration in liquid form, the oral drug components can be combined with any oral, non-toxic, pharmaceutically acceptable inert carrier such as ethanol, glycerol, water and the like. Moreover, when desired or necessary, suitable binders, lubricants, disintegrating agents and coloring agents can also be incorporated into the mixture. Suitable binders include starch, gelatin, natural sugars such as glucose or beta-lactose, corn-sweeteners, natural and synthetic gums such as acacia, tragacanth or sodium alginate, carboxymethylcellulose, polyethylene glycol, waxes and the like.

Lubricants used in these dosage forms include sodium oleate, sodium stearate, magnesium stearate, sodium benzoate, sodium acetate, sodium chloride and the like.

Disintegrators include, without limitation, starch methyl cellulose, agar, bentonite, xanthan gum and the like.

Typical uncoated tablet cores suitable for administration of thrombin inhibitors are comprised of, but not limited to, the following amounts of standard ingredients:

| Excipient | General Range (%) | Preferred Range (%) | Most Preferred Range (%) |
|---|---|---|---|
| mannitol | 10-90 | 25-75 | 30-60 |
| microcrystalline | 10-90 | 25-75 | 30-60 |
| cellulose magnesium stearate | 0.1-5.0 | 0.1-2.5 | 0.5-1.5 |

Mannitol, microcrystalline cellulose and magnesium stearate may be substituted with alternative pharmaceutically acceptable excipients.

The compounds of the present invention can also be co-administered with suitable antiplatelet agents, including, but not limited to, fibrinogen receptor antagonists (e. g. to treat or prevent unstable angina or to prevent reocclusion after angioplasty and restenosis), anticoagulants such as aspirin, thrombolytic agents such as plasminogen activators or streptokinase to achieve synergistic effects in the treatment of various vascular pathologies, or lipid lowering agents including antihypercholesterolemics (e. g. HMG CoA reductase inhibitors such as lovastatin, HMG CoA synthase inhibitors, etc.) to treat or prevent atherosclerosis. For example, patients suffering from coronary artery disease, and patients subjected to angioplasty procedures, would benefit from coadministration of fibrinogen receptor antagonists and thrombin inhibitors of the present invention. Also, compounds of the present invention enhance the efficiency of tissue plasminogen activator-mediated thrombolytic reperfusion. Compounds of the present invention may be administered first following thrombus formation, and tissue plasminogen activator or other plasminogen activator is administered thereafter.

Typical doses of thrombin inhibitors of the present invention in combination with other suitable anti-platelet agents, anticoagulation agents, or thrombolytic agents may be the same as those doses of thrombin inhibitors administered without coadministration of additional anti-platelet agents, anticoagulation agents, or thrombolytic agents, or may be substantially less that those doses of thrombin inhibitors administered without coadministration of additional anti-platelet agents, anticoagulation agents, or thrombolytic agents, depending on a patient's therapeutic needs.

Compounds of formula (I) and their prodrugs as well as their intermediates and reagents can be prepared as set forth below. The various routes and examples for the synthesis of the compounds of the present invention are non-limiting. If they are neither commercially available nor subsequently described explicitly, they can be obtained by analogy to the strategies and examples described hereinafter, or by conventional synthetic procedures.

Some abbreviations that may appear in this application are as follows.

| Desigination | |
|---|---|
| AC₂O | Acetic anhydride |
| bs | Broad singlet |
| Boc (or BOC) | *tert*-Butoxycarbonyl |
| Boc₂O | Di-*tert*-Butyldicarbonate |
| DAST | Diethylaminosulfurtrifluoride |
| DCE | 1,2-Dichloroethane |
| DCM | Dichloromethane |
| DMAP | 4-Dimethylaminopyridine |
| DMF | N, N-Dimethylformamide |
| Et₂O | Diethylether |
| Et₃N | Triethylamine |
| EtOAc | Ethyl acetate |
| EtOH | Ethanol |
| HPLC | High pressure liquid chromatography |
| ⁱPrOH | Isopropanol |
| MCPBA | meta-Chloroperbenzoic acid |
| MsCl | Methanesulfonyl chloride |
| OGr | Organic leaving group based on oxygen |
| PG | Protecting group |
| PPh₃ | Triphenylphosphine |
| rt | Retention time |
| Tf₂O | Trifluoromethanesulfonic anhydride |
| TFA | Trifluoroacetic acid |
| TFAA | Trifluoroacetic acid anhydride |
| THF | Tetrahydrofuran |
| TLC | Thin layer chromatography |

The compounds of formula (I) of the present invention can be prepared starting from 2-amino-pyrimidin-4-yl-acetic acid ester intermediates such as those of formula (II) using the method shown in Scheme A for 2-[2-(5-Chloro-4-ethoxycarbonylmethyl-pyrimidin-2-ylamino)-ethyl]-piperidine-1-carboxylic acid tert-butyl ester.

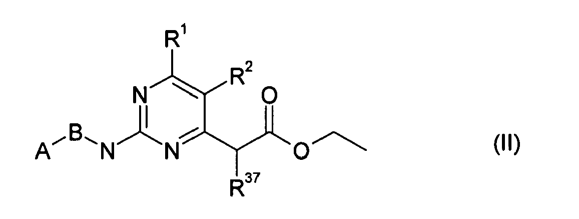

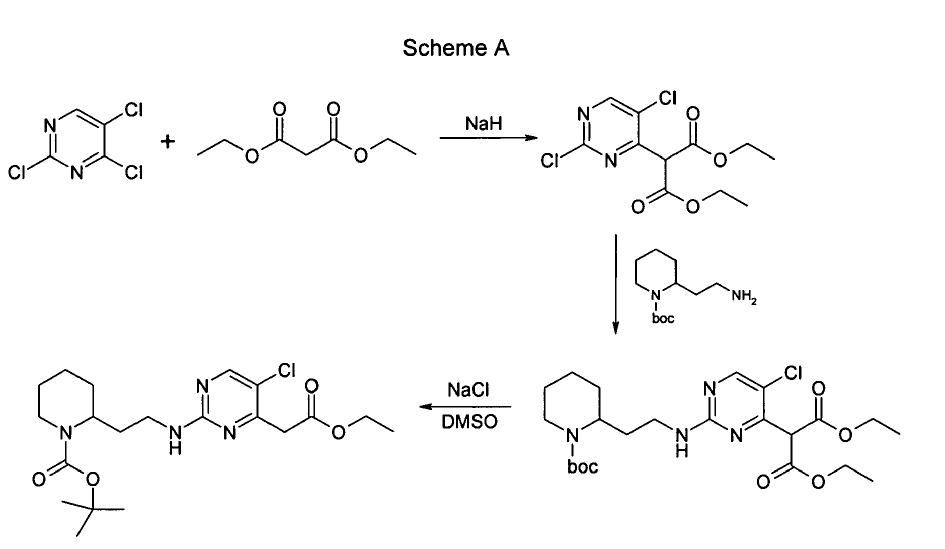

In the first step diethylmalonate is deprotonated and the formed enolate is reacted with 2,4,5-trichloropyrimidine giving rise to 4-malonate substituted 2,5-dichloropyrimidine in analogy to a procedure reported in *J. Heterocycl. Chem.,* **1980,** 589-592.

The chloro substituent in the 2 position of the pyrimidine core structure can be substituted in the second step by reaction with the Boc-protected 2-piperidin-2-yl-ethylamine, in analogy to a procedure shown in *Synth. Comm.,* 2000, 3171-3180.

Dealkoxycarbonylation was performed using sodium chloride in DMSO in the presence of traces of water according to a method reported in the literature (*Synthesis,* **1982,** 805-822; *JACS* 1998, 3402-3410).

In order to achieve the corresponding pyrimidine-oxides, oxidation of the pyrimidine has preferably to be performed at an earlier stage in the synthetic route to achieve a selective oxidation on the pyrimidine ring.

3-(Aminomethyl)-5-chloro-1H-indazole as precursors for the compounds of formula (I) may be conveniently prepared as illustrated in Scheme B and reported in *Synthetic Comm.* **1988,** 259-264 and literature cited therein.

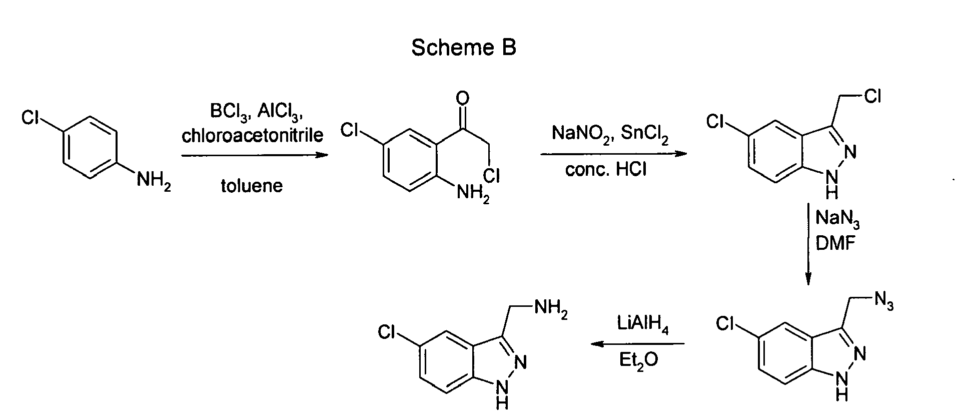

In the first step 1-(2-amino-5-chloro-phenyl)-2-chloro-ethanone was prepared from 4-chloro-phenylamine in a Friedel-Crafts acylation with chloroacetonitrile. The product was submitted to diazotization and cyclization to give 5-chloro-3-chloromethyl-1H-indazole.

In the third step treatment with NaN₃ affords 3-azidomethyl-5-chloro-1H-indazole, which is reduced in a final step with LiAIH₄.

**General procedure for making compounds of the invention**

In general, compounds having the structure (I) 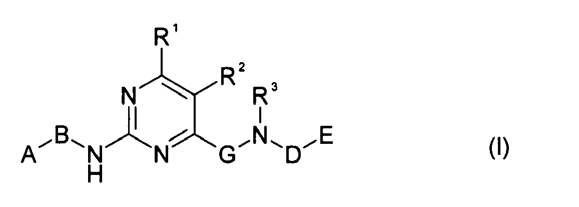 wherein the variables have the above described meanings, may be prepared as shown in Scheme D. 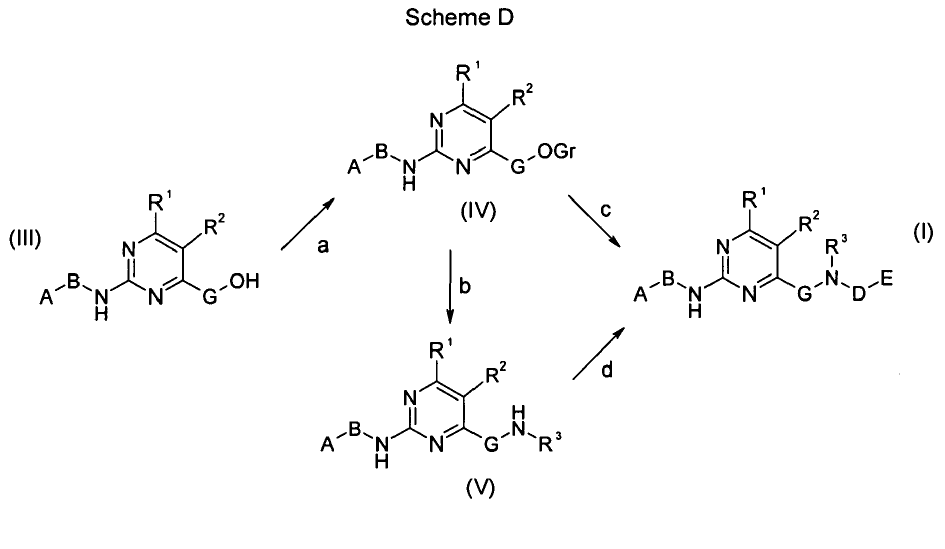 Starting materials for the synthesis of the claimed compounds (I) may be prepared by lithium borohydride reduction of the corresponding acetic acid ester intermediates (II) according to Scheme E (see e.g. Bioorg. Med. Chem. Lett. 2003, 1483-1486; S. D. Burke, R. L. Danheiser, *Handbook of Reagents for Organic Synthesis, Oxidizing and Reducing Agents;* John Wiley and Sons: New York, **1999**, 209-212; M. Fieser, *Reagents for Organic Synthesis;* John Wiley and Sons: New York, **1989**, *Vol. 14*, 191; K. Soai, A. Ookawa, J. Org. Chem. **1986,** 51, 4000-4005; H. C. Brown, E. J. Mead, B. C. S. Rao, J. *Am. Chem. Soc.* **1955**, 75, 6209-6213.).

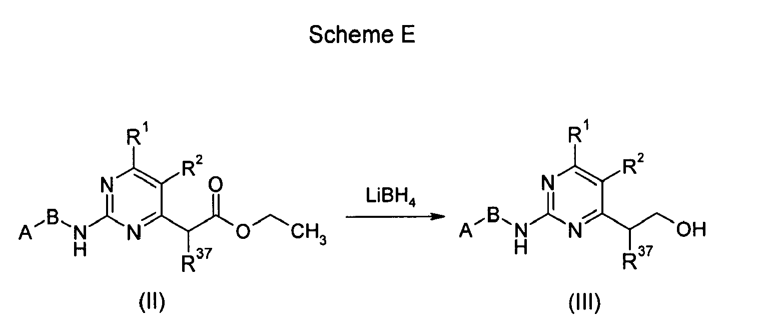

The alcohol function of the resulting compounds (III) has to be transformed in the next step into a reactive leaving group (OGr = e.g. mesylate, p-tosylate) to enable the subsequent nucleophilic displacement with an amine (path a). This transformation may be performed using suitably substituted sulfonyl halides, e.g. methylsulfonyl chloride or para-toluenesulfonyl chlorid (see e.g. C. Kaes, M. Hosseini, A. de Cian, J. Fischer, *Tetrahedron Lett.* **1977,** 22, 3901-3904;K. T. Potts, D. A. Usifer, A. Guadalupe, H. D. Abruna, J. *Am. Chem. Soc.* 1987, 13, 3961-3967.)

Compounds (IV) can than be reacted in the fashion of a nucleophilic substitution reaction with primary or secondary amines yielding intermediates (V) (path b) or final compounds (I) (path c). Methods referring to path b and c are described in e.g. T. N. Lambert, S. Chittamuru, H. K. Jacobs, A. S. Gopalan, *Tetrahedron Lett.* **2002,** 43, 7379-7383.

Secondary amino compounds (V) can be further reacted with acids or acid halides to form amides (according to methods described in T. J. Reddy, L. Chan, N. Turcotte, M. Proulx, O. Z. Pereira, S. K. Das, A. Siddiqui, W. Wang, C. Poisson, C. G. Yannopoulos, D. Bilimoria, *Bioorg. Med. Chem. Lett.* **2003,** *19,* 3341-3344; W. H. Miller, M. A. Seefeld, K. A. Newlander, C. C. K. Yuan, M. S. Head, D. J. Payne, S. F. Rittenhouse, T. D. Moore, J. *Med. Chem.* 2002, 15, 3246-3256 or with sulfonyl halides to yield sulfonamides according to methods described in E. Takashiro, Y. Nakamura, S. Miyamoto, Y. Ozawa, A. Sigiyama, K. Fujimoto, *Bioorg. Med. Chem.* **1999,** 9, 2105-2114; M. de Kort, A. W. Tuin, S. Kuiper, H. S. Overkleeft, G. A. van der Marel, R. C. Buijsman, *Tetrahedron Lett.* **2004,** 10, 2171-2176 or with aldehydes in a reductive amination procedure (see e.g. A. F. Abdel-Magid, K. G. Carson, B. D. Harris, C. A. Maryanoff, R. D. Shah, J. *Org. Chem.* 1996, 61, 3849-3862 to give tertiary amines of the common formula (I) (path d).

### PREPARATIONS

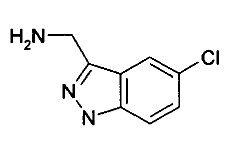

### Step 1

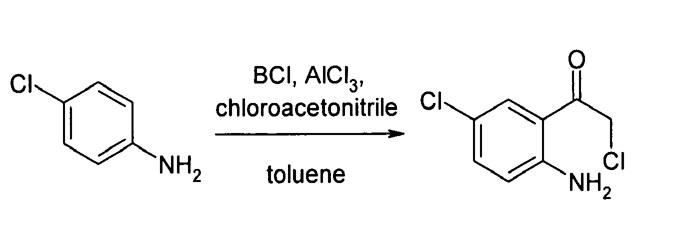

### 5-chloro-2-amino-a-chloroacetophenone

To a stirred solution of boron trichloride (8,62 mL of 1 M solution in heptane) in dry benzene (5 mL), a solution of 4-chloroaniline (1,00 g, 7,84 mmol) in dry benzene (15 mL) is added dropwise under ice cooling. To the resulting mixture containing 4-chloroaniline borontrichloride complex, chloroacetonitrile (0.60 mL, 9,41 mmol) and aluminiumtrichloride (1,15 g, 8,62 mmol) are added successively. The mixture is then refluxed for 6 h under nitrogen, becoming a solution of two layers. The evolved hydrogen chloride is absorbed through a drying tube containing silica gel or calcium chloride to a surface of aqueous sodium hydroxide. After cooling, ice 2 N hydrochloric acid is added and a yellow precipitate is formed. To hydrolyze the ketimine of 5-chloro-2-amino-a-chloroacetophenone, the mixture is warmed at 80 °C under stirring, until the precipitate has dissolved (ca. 30 min). The cooled mixture is extracted with chloromethane (three times) and the organic layer is washed with water, dried with sodium sulfate, and concentrated. The neutral fraction obtained (1,00 g) is recrystallized to obtain 680 mg (3,33 mmol, 43% yield) of pure 5-chloro-2-amino-a-chloroacetophenone.
LC/MS (I) (5-95%, 5 min): 2.77, 245 (M+H+AcCN).

### Step 2

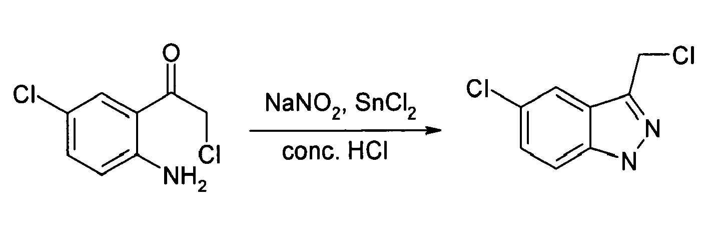

### 5-chloro-3-(chloromethyl)-1H-indazole

To a stirred suspension of 2-amino-5-chloro-α-chloroacetophenone (670 mg, 3,28 mmol) in conc. hydrochloric acid (10 mL) is added a solution of sodium nitrite (250 mg, 3,61 mmol) in water (2 mL) while maintaining the reaction temperature at 0 °C. After 1 h a solution of SnCl₂•2H2O (1,78 g, 7,87 mmol) in conc. hydrochloric acid (5 mL) is added to the reaction mixture, which is then stirred at the same temperature for 1 h. Next, ice water is added to the reaction mixture. The precipitate is collected by filtration, washed with water and dried giving crude 5-chloro-3-(chloromethyl)-1H-indazole (370 mg, 1,84 mmol, 56% yield) which is used in the next step without further purification.
LC/MS (I) (5-95%, 5 min): 2.67, no mass peak.

Step 3

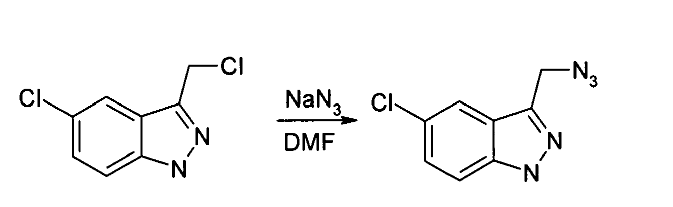

### 3-(azidomethyl)-5-chloro-1H-indazole

A stirred solution containing 5-chloro-3-(chloromethyl)-1H-indazole (370 mg, 1,84 mmol), sodium azide (156 mg, 2,40 mmol), water (0,5 mL) and DMF (5,00 mL) is warmed at 90 °C for 1 h and then the mixture is concentrated under reduced pressure. Ice is added and the resulting precipitate is collected by filtration and washed with water giving 330 mg (1,59 mmol, 85% yield) of 3-(azidomethyl)-5-chloro-1H-indazole.
LC/MS (I) (5-95%, 5 min): 2.63, 249 (M+H+AcCN).

### Step 4

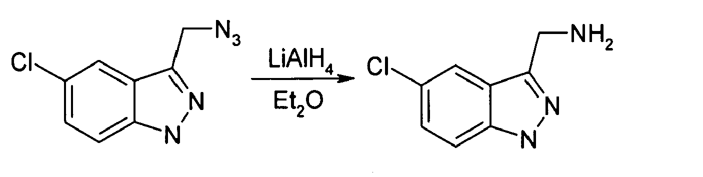

### 3-(aminomethyl)-5-chloro-1H-indazole

To a stirred 1M THF-solution of LiAIH₄ (5,00 mL) is added a solution of 3-(azidomethyl)-5-chloro-1H-indazole (330 mg ,1,59 mmol) in Et₂O (10 mL) dropwise at room temperature, and the mixture is refluxed for 1 h. After quenching the excess of LiAlH₄ with wet Et₂O the precipitate is filtered off and washed with DCM-EtOH (9:1), giving crude 3-(aminomethyl)-5-chloro-1H-indazole. The purification by column chromatography (silica gel, eluent = 10% DCM in methanol with 0.1 % Et₃N) affords 105 mg (0,58 mmol, 37%) of pure material. ¹H NMR (d6-DMSO, 200 MHz): 4.01 (s, 2H), 7.25-7.28 (m, 1 H), 7.43-7.47 (m, 1 H), 7.92-7.93 (m, 1H).
LC/MS (I) (5-95%, 5 min): 1.59, 182 (M+H).

### Example 1

### Step 1

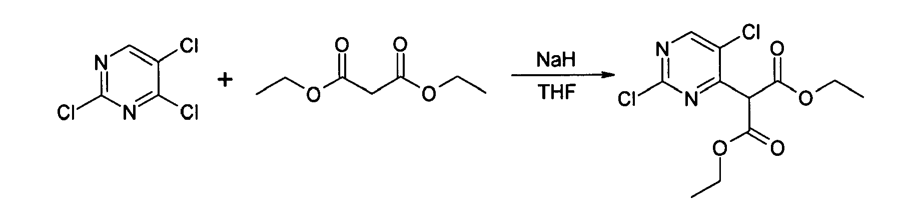

### 2-(2,5-Dichloro-pyrimidin-4-yl)-malonic acid diethyl ester

To a solution of 0.87 g (5.45 mmol) of diethylmalonate in 6 mL THF is added 0.27 g (6.54 mmol) sodium hydride and another 6 mL THF. The reaction mixture is stirred 1 h at room temperature and 1 g (5.45 mmol) 2,4,5-trichloropyrimidine in 8 mL THF is added. The mixture is stirred 1 h by room temperature and then 4 h at 60 °C.
The mixture is allowed to cool down to room temperature and washed with a saturated sodium bicarbonate solution, water and brine and dried over sodium sulfate. The solvent is evaporated under reduced pressure and the crude material is purified by column chromatography (silica gel, eluent = 10% EtOAc in hexane) giving 73% yield.
LC/MS (I) (5-95%, 5 min): 2.75, 307 (M+H).

### Step 2

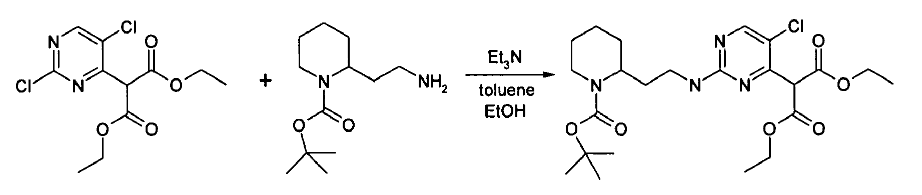

### 2-{2-[2-(1-tert-Butoxycarbonyl-piperidin-2-yl)-ethylamino]-5-chloro-pyrimidin-4-yl}-malonic acid diethyl ester

2-(2,5-Dichloro-pyrimidin-4-yl)-malonic acid diethyl ester (1.25 g, 4.07 mmol), 2-(2-amino-ethyl)-piperidine-1-carboxylic acid tert-butyl ester (0.93 g, 4.07 mmol) and 0.85 mL (6.11 mmol) triethylamine are dissolved in 8 mL toluene and 2 mL ethanol. The solution is heated in a bomb tube by 120 °C overnight. The mixture is allowed to cool down to room temperature and washed with a saturated sodium bicarbonate solution, water and brine and dried over sodium sulfate. The solvent is evaporated under reduced pressure and the crude material is used without further purification in the next step.
NMR (d6-DMSO, 200 MHz): 1.23 (t, 6H), 1.35 (s, 9H), 1.43-1.70 (m, 7H), 1.80-1.98 (m, 1H), 2.70-2.78 (m, 1H), 3.05-3.22 (m, 2H), 3.75-3.90 (m, 1H), 3.98-4.08 (m, 1H), 4.17 (q, 4H), 4.98 (s, 1H), 8.28 (s, 1 H).
LC/MS (I) (5-90%, 5 min): 3.79, 499 (M+H).

### Step 3

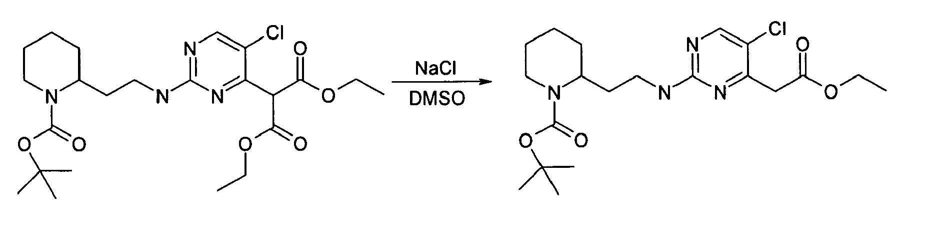

### 2-[2-(5-Chloro-4-ethoxycarbonylmethyl-pyrimidin-2-ylamino)-ethyl]-piperidine-1-carboxylic acid tert-butyl ester

2-{2-[2-(1-tert-Butoxycarbonyl-piperidin-2-yl)-ethylamino]-5-chloro-pyrimidin-4-yl}-malonic acid diethyl ester (1.05 mg, 2.10 mmol) and sodium chloride (123 mg, 2.10 mmol) are dissolved in 21 mL DMSO and 0.21 mL water are is added. The reaction is stirred 2h at 160 °C and after cooling down to room temperature brine is added. The aqueous layer is extracted with ethyl acetate and the organic phase is washed with a saturated sodium bicarbonate solution, water and brine and dried over sodium sulfate. The solvent is evaporated under reduced pressure. The crude product is purified by column chromatography (silica gel, eluent = 10% EtOAc in hexane) giving 19% yield. NMR (d6-DMSO, 200 MHz): 1.21 (t, 3H), 1.23-1.34 (m, 1H), 1.35 (s, 9H), 1.41-1.70 (m, 6H), 1.80-1.95 (m, 1H), 2.70-2.78 (m, 1H), 3.10-3.20 (m, 2H), 3.70 (s, 2H), 3.78-3.85 (m, 1 H), 4.08 (q, 2H), 4.10-4.20 (m, 1 H), 7.18-7.24 (s, 1 H), 8.22 (s, 1H). LC/MS (II) (5-90%, 5 min): 3.58, 327 (M+H).

### Step 4

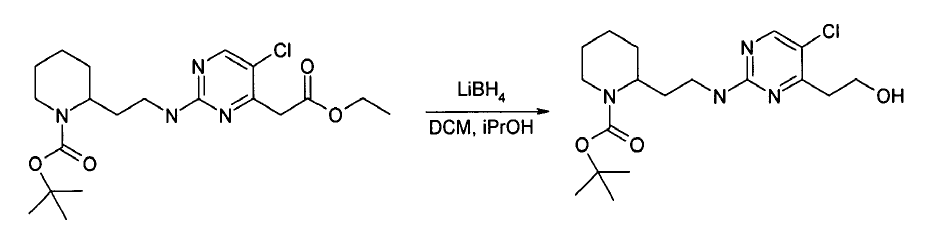

### 2-{2-[5-Chloro-3-fluoro-4-(2-hydroxy-ethyl)-pyridin-2-ylamino]-ethyl}-piperidine-1-carboxylic acid tert-butyl ester

To a solution of 50.0 mg (0.12 mmol) of 2-[2-(5-chloro-4-ethoxycarbonylmethylpyrimidin-2-ylamino)-ethyl]-piperidine-1-carboxylic acid tert-butyl ester in 0.80 mL DCM and 0.20 mL iPrOH is added 129 µL (0.257 mmol) of a 2M lithium borohydride solution in tetrahydrofuran by 0 °C and the resulting mixture is stirred for 2 h at room temperature. After addition of 20 mL of methanol the mixture is stirred until gas evolution has ceased. The solvent is evaporated under reduced pressure to give the product in 43% yield.
NMR (d6-DMSO, 200 MHz): 1.15-1.25 (m, 2H), 1.34 (s, 9H), 1.43-1.70 (m, 5H), 1.86-1.94 (m, 1 H), 2.72-2.81 (m, 3H), 3.10-3.20 (m, 2H), 3.71 (q, 2H), 3.75-3.90 (m, 1H), 4.10-4.22 (m, 1 H), 4.57-4.60 (m, 1H), 7.02-7.07 (m, 1H), 8.12 (s, 1H).
LC/MS (1) (5-95%, 5 min): 2.77, 385 (M+H).

### Step 5

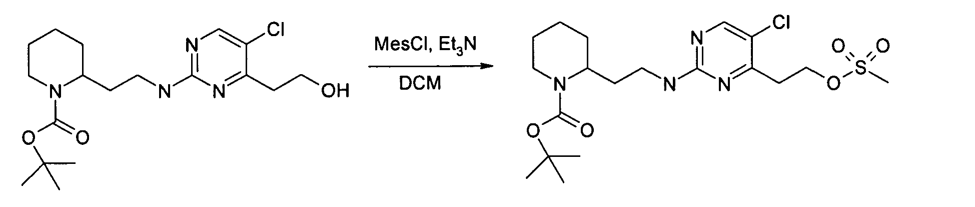

### 2-{2-[5-Chloro-4-(2-methanesulfonyloxy-ethyl)-pyrimidin-2-ylamino]-ethyl}-piperidine-1-carboxylic acid tert-butyl ester

2-{2-[5-Chloro-3-fluoro-4-(2-hydroxy-ethyl)-pyridin-2-ylamino)-ethyl}-piperidine-1-carboxylic acid tert-butyl ester (20.0 mg, 52.23 µmol) are dissolved in 1 mL dry DCM by 0 °C. Triethylamine (16.0 µL, 114 µmol) and a solution of 16.0 µL (115 µmol) methane sulfonic acid chloride in 0.2 mL DCM are added and the solution is stirred 1 h at 0 °C. To the solution DCM was added and the organic phase is washed with a saturated sodium bicarbonate solution, water and brine and dried over sodium sulfate. The crude product is used without further purification.

### Step 6

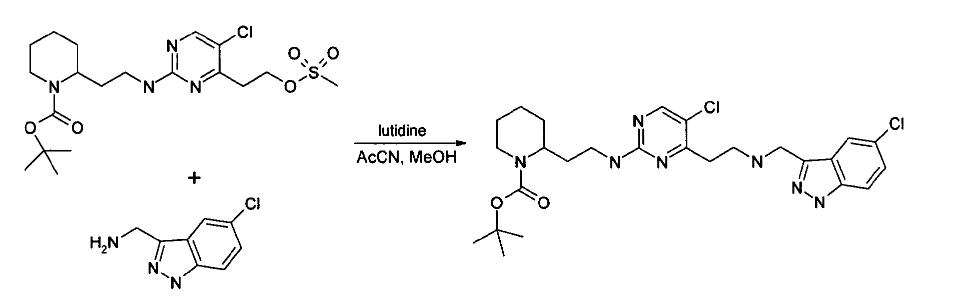

### 2-[2-(5-Chloro-4-{2-[(5-chloro-1H-indazol-3-ylmethyl)-amino]-ethyl}-pyrimidin-2-ylamino)-ethyl]-piperidine-1-carboxylic acid tert-butyl ester

To a solution of 24.0 mg (52.0 mmol) of crude 2-{2-[5-Chloro-4-(2-methanesulfonyloxyethyl)-pyrimidin-2-ylamino]-ethyl}-piperidine-1-carboxylic acid tert-butyl ester in 1 mL of acetonitrile are added 12.2 µL (0.104 mmol) of lutidine and 10 mg (0.057 mmol) of (5-chloro-1 H-indazol-3-yl)-methylamine. To the solution 0.5 mL MeOH are added and the reaction mixture is stirred overnight at 60 °C and 5h at reflux. The solvent is evaporated under reduced pressure and after column chromatography the pure material is isolated in 32% yield.
LC/MS (I) (5-95%, 5 min): 2.78, 548 (M+H).

### Step 7

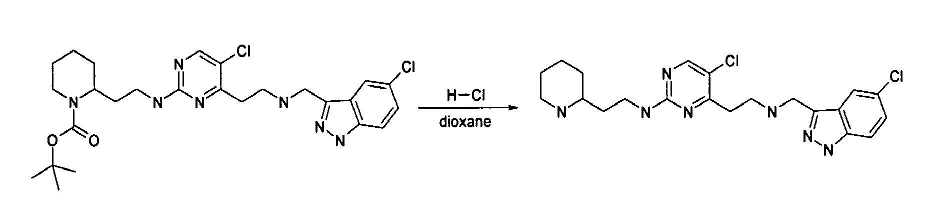

### (5-Chloro-4-{2-[(5-chloro-1H-indazol-3-ylmethyl)-aminol-ethyl}-pyrimidin-2-yl)-(2-piperidin-2-yl-ethyl)-amine

To a solution of 9.00 mg (16.0 mmol) of 2-[2-(5-Chloro-4-{2-[(5-chloro-1H-indazol-3-ylmethyl)-amino]-ethyl}-pyrimidin-2-ylamino)-ethyl]-piperidine-1-carboxylic acid tert-butyl ester in 0.5 mL dioxane is added 500 µL of a 2M hydrochloric acid solution in dioxane. The solution is stirred 3 h at room temperature. The solvent is evaporated by reduced pressure and the crude product is recrystallized by Et₂O yielding 6.0 mg (72% yield) of (5-chloro-4-{2-[(5-chloro-1 H-indazol-3-ylmethyl)-amino]-ethyl}-pyrimidin-2-yl)-(2-piperidin-2-yl-ethyl)-amine.
LC/MS (I) (5-60%, 10 min): 3.73, 448 (M+H).
NMR (d6-DMSO, 200 MHz): 1.10-2.08 (m, 8H), 2.18-2.26 (m, 1 H), 2.72-2.87 (m, 1 H), 2.96-3.10 (m, 1 H), 3.29-3.60 (m, 4H), 4.08-4.13 (m, 2H), 4.62 (s, 2H), 7.35-7.38 (m, 1 H), 7.57-7.60 (m, 1 H), 8.15 (s, 1 H), 8.21 (s, 1H).
LC/MS (I) (10-60%, 12 min): 3.73, 448 (M+H).

Following the procedure outlined for steps 5-7 in Example 1, the compounds listed in the Table 1 were prepared. Purification was performed by HPLC chromatography.

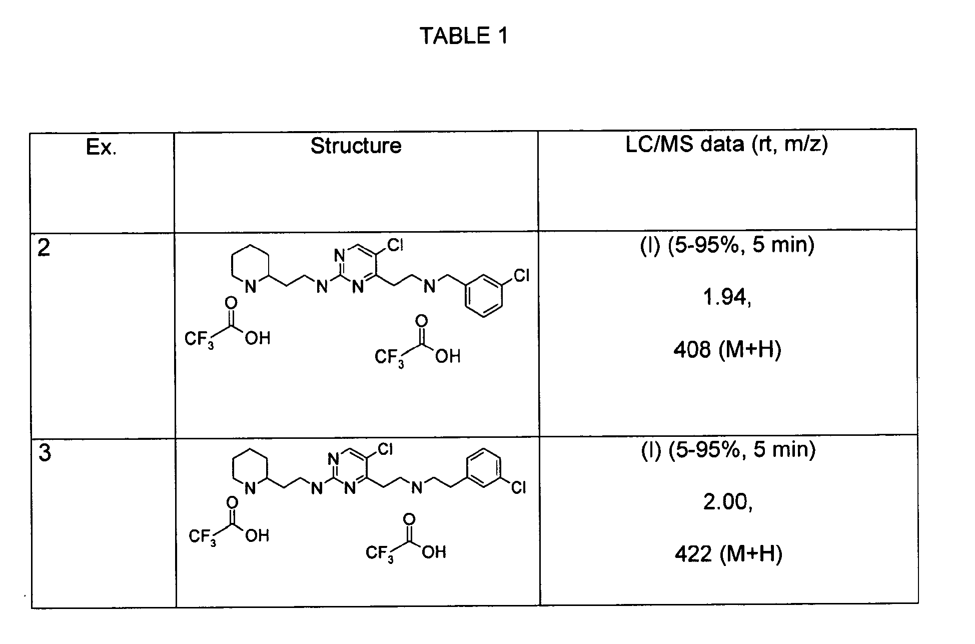

### Example 4 Kᵢ determinations thrombin

The Ki determinations were carried out at 20 °C with the fluorogenic substrate Tosyl-GPR-AMC (Bachem, Heidelberg, Germany; λ_{exc} = 370 nm, λₑₘ = 450 nm) at a thrombin concentration of 100 pM in HBS pH 7.4. The substrate was added to a final concentration of 20 µM in a total assay volume of 100 µl. The enzymatic reaction was started by addition of substrate. The emission at 450 nm was monitored in 1 minute intervals for 10 minutes using a polarstar reader (BMG Laboratories, Offenburg, Germany). Initial velocities of the control and the inhibited reactions (vₒ and vᵢ) were estimated in FU/min at different compound concentrations. The inhibition constants were calculated using the Michaelis-Menten equation for competitive inhibition.

HBS: 10 mM Hepes, 150 mM NaCl, 0.005 % Tween20, pH 7.4

The Ki values for the compounds 1-3 are ≤ 10 µM.

### Example 5 aPTT protocol

The aPTT measurements are carried out with an CoaData coagulometer from HelenaBioscience on 50ul human standard plasma obtained from Dade Behring. After activation with 50ul ellagic acid and cephalin using the Actin kit from Dade Behring, coagulation is triggered by addition of 50ul 25mM calcium chloride. Clotting time is measured by the instrument in seconds.

### Example 6 Protease assays

### Factor Xa:

The Ki determinations are carried out at 20 °C with the fluorogenic substrate Boc-LGR-AMC (Bachem, Heidelberg, Germany; λ_{exc} = 370 nm, λₑₘ = 450 nm) at a fXa concentration of 1 nM in HBS pH 7.4, 5 mM CaCl₂. The substrate is added to a final concentration of 100 µM in a total assay volume of 100 µl. The enzymatic reaction is started by addition of substrate. The emission at 450 nm is monitored in 1 minute intervals for 10 minutes using a polarstar reader (BMG Laboratories, Offenburg, Germany). Initial velocities of the control and the inhibited reactions (vₒ and vᵢ) are estimated in FU/min at different compound concentrations. The inhibition constants are calculated using the Michaelis-Menten equation for competitive inhibition.

HBS: 10 mM Hepes, 150 mM NaCl, 0.005 % Tween20, pH 7.4

### Tryptase:

The Ki determinations are carried out at 20 °C with the fluorogenic substrate Boc-FSR-AMC (Bachem, Heidelberg, Germany; λ_{exc} = 370 nm, λₑₘ = 450 nm) at a Tryptase concentration of 1 nM in HBS pH 7. The substrate is added to a final concentration of 20 µM in a total assay volume of 100 µl. The enzymatic reaction is started by addition of substrate. The emission at 450 nm is monitored in 1 minute intervals for 10 minutes using a polarstar reader (BMG Laboratories, Offenburg, Germany). Initial velocities of the control and the inhibited reactions (vₒ and vᵢ) are estimated in FU/min at different compound concentrations. The inhibition constants are calculated using the Michaelis-Menten equation for competitive inhibition.

HBS: 10 mM Hepes, 150 mM NaCl, 0.005 % Tween20, pH 7

### Trpysin:

The Ki determinations are carried out at 20 °C with the fluorogenic substrate Z-GGR-AMC (Bachem, Heidelberg, Germany; λ_{exc} = 370 nm, λₑₘ = 450 nm) at a Trypsin concentration of 0.001 U/ml in TBS pH 8. The substrate is added to a final concentration of 100 µM in a total assay volume of 100 µl. The enzymatic reaction is started by addition of substrate. The emission at 450 nm is monitored in 1 minute intervals for 10 minutes using a polarstar reader (BMG Laboratories, Offenburg, Germany). Initial velocities of the control and the inhibited reactions (vₒ and vᵢ) are estimated in FU/min at different compound concentrations. The inhibition constants are calculated using the Michaelis-Menten equation for competitive inhibition.

TBS: 20 mM Tris, 150 mM NaCl, 0.005 % Tween20, pH 8

### Chymotrpysin:

The Ki determinations are carried out at 20 °C with the fluorogenic substrate H-AAF-AMC (Bachem, Heidelberg, Germany; λ_{exc} = 370 nm, λₑₘ = 450 nm) at a Chymotrpysin concentration of 1 nM in TBS pH 8. The substrate is added to a final concentration of 100 µM in a total assay volume of 100 µl. The enzymatic reaction is started by addition of substrate. The emission at 450 nm is monitored in 1 minute intervals for 10 minutes using a polarstar reader (BMG Laboratories, Offenburg, Germany). Initial velocities of the control and the inhibited reactions (vₒ and vᵢ) are estimated in FU/min at different compound concentrations. The inhibition constants are calculated using the Michaelis-Menten equation for competitive inhibition.

TBS: 20 mM Tris, 150 mM NaCl, 0.005 % Tween20, pH 8

### Elastase

The Ki determinations are carried out at 20 °C with the fluorogenic substrate MeOSuc-AAPV-AMC (Loxo, Heidelberg, Germany; λ_{exc} = 370 nm, λₑₘ = 450 nm) at an Elastase concentration of 5 nM in Hepes buffer pH 7. The substrate is added to a final concentration of 100 µM in a total assay volume of 100 µl. The enzymatic reaction is started by addition of substrate. The emission at 450 nm is monitored in 1 minute intervals for 10 minutes using a polarstar reader (BMG Laboratories, Offenburg, Germany). Initial velocities of the control and the inhibited reactions (vₒ and vᵢ) are estimated in FU/min at different compound concentrations. The inhibition constants are calculated using the Michaelis-Menten equation for competitive inhibition.

### Hepes buffer: 10 mM Hepes, 50 mM NaCl, 0.005 % Tween20, pH 7

### Plasmin

The Ki determinations are carried out at 20 °C with the fluorogenic substrate H-D-ALK-AMC (Bachem, Heidelberg, Germany; λ_{exc} = 370 nm, λₑₘ = 450 nm) at a plasmin concentration of 1 nM in HBS pH 7.4, 5 mM CaCl2. The substrate is added to a final concentration of 100 µM in a total assay volume of 100 µl. The enzymatic reaction is started by addition of substrate. The emission at 450 nm is monitored in 1 minute intervals for 10 minutes using a polarstar reader (BMG Laboratories, Offenburg, Germany). Initial velocities of the control and the inhibited reactions (vₒ and vᵢ) are estimated in FU/min at different compound concentrations. The inhibition constants are calculated using the Michaelis-Menten equation for competitive inhibition.

HBS: 10 mM Hepes, 150 mM NaCl, 0.005 % Tween20, pH 7.4

## Claims

1. A compound of formula (I) 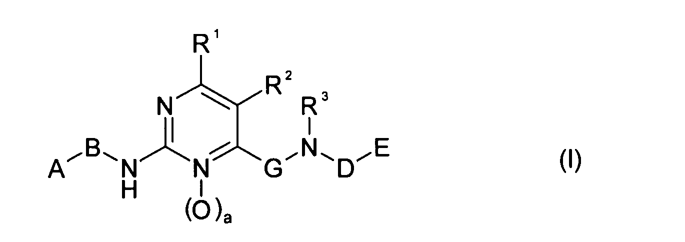 or a pharmaceutically acceptable salt thereof, wherein:
a is 0 or 1;
R¹ is hydrogen;
halogen;
CN;
CNO; or
C₁₋₄alkyl, optionally substituted with one or more fluoro;
R² is hydrogen;
halogen;
CN;
CNO;
C₁₋₆alkyl, optionally substituted with one or more fluoro;
C₃₋₆cycloalkyl, optionally substituted with one or more fluoro; or
O-C₁₋₄ alkyl, optionally substituted with one or more fluoro;
R³ is hydrogen;
C₁₋₄alkyl, optionally substituted with one or more fluoro; or
C₃₋₆ cycloalkyl, optionally substituted with one or more fluoro;
A is A¹, wherein A¹ is selected from the group consisting of:
phenyl;
naphthyl;
heterocycle containing 1, 2, 3 or 4 heteroatoms, which are the same or different and
selected from the group consisting of -O-, -S-, -S(O)-, -S(O₂)- -N=,
-N(O)= and -N(R⁴)-; and
heterobicycle containing 1, 2, 3, 4, 5 or 6 heteroatoms, which are the same or different
and selected from the group consisting of -O-, -S-, -S(O)-, -S(O₂)-, -N=,
-N(O)= and -N(R⁴)-;
wherein A¹ is optionally substituted with one or independently from each other more of
A²;
A³;
halogen;
CN;
CNO;
-N(R⁵R⁶);
-OH;
=O, where the ring is at least partially saturated;
C₃₋₆ cycloalkyl;
-COOR⁷; or
-CONR⁸R⁹;
-S(O)₂NR^{8a}R^{9a}
and wherein R⁴, R⁵, R⁶ are independently selected from the group consisting of R⁷a, -C(O)-R⁷a, -C(O)O-R^{7a}, -C(O)NR^{7a}R^{7b}, -S(O)₂NR^{7a}R^{7b}, and S(O)₂-R^{7a};
and wherein R⁷, R^{7a}, R^{7b}, R⁸, R^{8a}, R⁹, R^{9a} are independently hydrogen or C₁₋₄ alkyl, wherein each C₁₋₄ alkyl is optionally substituted with one or more substituents independently selected from the group consisting of -COOH; -COO-C₁₋₄ alkyl; -OH; -NH₂; -NH-C₁₋₄ alkyl; -N(C₁₋₄ alkyl)₂; and C₃₋₆ cycloalkyl;
Optionally R⁴ is a bond to directly attach A to B;
A² is selected from the group consisting of A⁴, -O-A⁴ and -N(R¹⁰)-A⁴,
wherein A⁴ is phenyl or a heterocycle containing 1, 2, 3 or 4 heteroatoms, which are the same or different and selected from the group consisting of -O-, -S-, - S(O)-, -S(O₂)-, -N=, -N(O)= and -N(R¹¹)-; wherein A⁴ is optionally substituted with one or independently from each other more of
fluoro;
chloro;
-N(R¹²R¹³)
C₁₋₄ alkyl or -O-C₁₋₄ alkyl, both optionally substituted with one or independently from each other more of fluoro or -N(R¹⁴R¹⁵);
and wherein R¹⁰, R¹², R¹³, R¹⁴, R¹⁵ are independently hydrogen or C₁₋₄ alkyl;
and wherein R¹¹ is selected from the group consisting of hydrogen, C₁₋₄ alkyl
and -C(O)-C₁₋₄ alkyl;
A³ is selected from the group consisting of C₁₋₆ alkyl, -O-C₁₋₆ alkyl and -N(R¹⁶)-C₁₋₆ alkyl, wherein the C₁₋₆ alkyl group is optionally substituted with one or independently from each other more of
fluoro;
-N(R¹⁷R¹⁸);
A⁵;
and/or A³ is optionally interrupted with one or more oxygen; and wherein R¹⁶, R¹⁷, R¹⁸ are independently hydrogen or C₁₋₄ alkyl;
A⁵ is phenyl or a heterocycle containing 1, 2, 3 or 4 heteroatoms, which are the same or different and selected from the group consisting of -O-, -S-, -S(O)-, -S(O₂)-, -N=, - N(O)= and -N(R¹⁹)-; wherein A⁵ is optionally substituted with one or independently from each other more of
fluoro;
chloro;
-N(R²⁰R²¹)
C₁₋₄ alkyl or -O-C₁₋₄ alkyl, both optionally substituted with one or independently from each other more of fluoro or -N(R²²R²³);
and wherein R¹⁹ is selected from the group consisting of hydrogen, C₁₋₄ alkyl and -C(O)-C₁₋₄ alkyl;
and wherein R²⁰, R²¹, R²², R²³ are independently hydrogen or C₁₋₄ alkyl;
B is selected from the group consisting of -Y-Z-; -Y-Z-C(O)-; -Y-Z-O-C(O)-; -Y-Z-S(O)₂-; and -Y-Z-NH-C(O)- wherein
Y is a bond, -O-, -S-, -N(R²⁴)-, -N(R²⁵)-C(O)-, -C(O)-N(R²⁶)-, or -C(O)-;
Z is C₁₋₆ alkyl,
optionally interrupted with oxygen, sulfur or -N(R²⁷)- and/or optionally substituted with one or independently from each other more of
halogen;
CN;
CNO;
C₃₋₆ cycloalkyl;
-COOR²⁸;
-CON(R²⁹R³⁰)
and/or optionally one chain carbon forms part of a C₃₋₆ cycloalkyl;
and wherein R²⁴, R²⁵, R²⁶, R²⁷ , R²⁸, R²⁹, R³⁰ are independently
hydrogen; or
C₁₋₄ alkyl, optionally substituted with -COOR³¹ or -CON(R³²R³³)
wherein R³¹, R³², R³³ are independently hydrogen or C₁₋₄ alkyl;
G is -CH(R³⁷)-C(R³⁸R³⁹)-;
-CH(R³⁷)-C(R⁴⁰R⁴¹)-C(R³⁸R³⁹)-;
wherein R³⁷, R⁴⁰, R⁴¹ are independently
hydrogen;
F;
C₁₋₄ alkyl, optionally substituted with one or more fluoro;
C₃₋₆ cycloalkyl, optionally substituted with one or more fluoro;
wherein R³⁸, R³⁹ are independently
hydrogen;
C₁₋₄ alkyl, optionally substituted with one or more fluoro;
C₃₋₆ cycloalkyl, optionally substituted with one or more fluoro; or R³⁸ and R³⁹, or R⁴⁰ and R⁴¹ or R³⁸ and R⁴⁰ form together C₃₋₆ cycloalkyl, optionally
substituted with one or more fluoro, -OH, C₁₋₄ alkyl;
or R³⁷ and R³⁸ in the case of -CH(R³⁷)-C(R³⁸R³⁹)-, or R³⁷ and R⁴⁰ in the case of -CH(R³⁷)-C(R⁴⁰R⁴¹)-C(R³⁸R³⁹)-, form together C₃₋₆ cycloalkyl, optionally
substituted with one or more fluoro, -OH, C₁₋₄ alkyl;
D is C₁₋₆ alkyl,
optionally interrupted with oxygen, sulfur or -N(R⁴²)-
and/or optionally substituted with halogen, CN; CNO; C₃₋₆ cycloalkyl;
and/or optionally one chain carbon or two vicinal carbons form part of a C₃₋₆ cycloalkyl, wherein R⁴² is selected from the group consisting of hydrogen, C₁₋₄ alkyl, C₃₋₆ cycloalkyl and -C(O)-C₁₋₄ alkyl;
E is E¹, wherein E¹ is selected from the group consisting of
phenyl;
naphthyl;
heterocycle containing 1, 2, 3 or 4 heteroatoms, which are the same or different and
selected from the group consisting of -O-, -S-, -S(O)-, -S(O₂)-, -N=,
-N(O)= and -N(R⁴³)-; and
heterobicycle containing 1, 2, 3, 4, 5 or 6 heteroatoms, which are the same or different
and selected from the group consisting of -O-, -S-, -S(O)-, -S(O₂)- -N=, -N(O)= and -N(R⁴⁴)-;
wherein E¹ is optionally substituted with one or independently from each other more of
E²;
E³;
halogen;
CN;
CNO;
-N(R⁴⁵R⁴⁶);
-OH;
=O where the ring is at least partially saturated;
C₃₋₆ cycloalkyl;
-COOR⁴⁷; or
-CONR⁴⁸R⁴⁹;
-S(O)₂NR^{48a}R^{49a};
and wherein R⁴³, R⁴⁴, R⁴⁵, R⁴⁶ R^{48a}, R^{49a} are independently selected from the group consisting of
hydrogen;
C₁₋₄ alkyl optionally substituted with -OH and/or F;
and -C(O)-C₁₋₄ alkyl optionally substituted with -OH and/or F;
and wherein R⁴⁷, R⁴⁸, R⁴⁹ are independently hydrogen or C₁₋₄ alkyl, optionally substituted with -OH and/or F;
E² is selected from the group consisting of E⁴, -C(O)-E⁴, -O-E⁴ and -N(R⁵⁰)-E⁴, wherein E⁴ is phenyl or heterocycle containing 1, 2, 3 or 4 heteroatoms, which are the same or different and selected from the group consisting of -O-, -S-, -S(O)-, -S(O₂)- -N=, -N(O)= and -N(R⁵¹)-; wherein E⁴ is optionally substituted with one or independently from each other more of
fluoro;
chloro;
cyano;
=O, where the ring is at least partially saturated;
-N(R⁵²R⁵³);
C₁₋₄ alkyl; or
-O-C₁₋₄ alkyl;
and wherein R⁵⁰, R⁵², R⁵³ are independently hydrogen or C₁₋₄ alkyl, optionally substituted with -OH;
and wherein R⁵¹ is selected from the group consisting of
hydrogen;
C₁₋₄ alkyl, optionally substituted with -OH; and
-C(O)-C₁₋₄ alkyl, optionally substituted with -OH;
E³ is selected from the group consisting of C₁₋₆ alkyl, -O-C₁₋₆ alkyl; -N(R⁵⁴)-C₁₋₆ alkyl, wherein E³ is optionally substituted with one or independently from each other more of
fluoro;
OH;
-N(R⁵⁵R⁵⁶);
E⁵;
and/or E³ is optionally interrupted with one or more oxygen;
and wherein R⁵⁴, R⁵⁵, R⁵⁶ are independently hydrogen or C₁₋₄alkyl, optionally substituted with -OH;
E⁵ is phenyl or heterocycle containing 1, 2, 3 or 4 heteroatoms, which are the same or different and selected from the group consisting of -O-, -S-, -S(O)-, -S(O₂)-, -N=, -N(O)= and -N(R⁵⁷)-; wherein E⁵ is optionally substituted with one or independently from each other more of
fluoro;
chloro;
cyano;
=O, where the ring is at least partially saturated;
-N(R⁵⁸R⁵⁹);
C₁₋₄ alkyl or
-O-C₁₋₄ alkyl;
and wherein R⁵⁷ is independently selected from the group consisting of hydrogen;
C₁₋₄ alkyl, optionally substituted with -OH; and
-C(O)-C₁₋₄ alkyl, optionally substituted with -OH;
and wherein R⁵⁸, R⁵⁹ are independently hydrogen or C₁₋₄ alkyl, optionally substituted with -OH.

2. A compound according to claim 1, wherein R¹ is hydrogen.

3. A compound according to claim 1 or 2, wherein R² is hydrogen, chloro, -CH₃, -CH₂₋CH₃, -CH₂-CH₂-CH₃, -CH₂-CH₂-CH₂-CH₃, -CH₂F, -CHF₂ or -CN.

4. A compound according to any one of the preceding claims, wherein R³ is hydrogen.

5. A compound according to any one of the preceding claims, wherein A¹ is phenyl or heterocycle containing 1, 2, 3 or 4 heteroatoms, which are the same or different and selected from the group consisting of -O-, -S-, -S(O)-, -S(O₂)-, -N=, -N(O)= and -N(R⁴)-, wherein R⁴ has the meaning as indicated in claim 1.

6. A compound according to claim 5, wherein A¹ is selected from the group consisting of phenyl, pyridine, pyridine-N oxide, piperidine, morpholine, and pyrrolidine.

7. A compound according to any of the preceding claims, wherein R⁴ is a bond, hydrogen, -COOC₁₋₄ alkyl, methyl, ethyl, 2-hydroxyethyl, -COOH, -CH₂-COOH, - CH₂-COO-C₁₋₄ alkyl or cyclopropylmethyl and wherein A¹ is optionally substituted with 1, 2, 3 or 4 F.

8. A compound according to any one of the preceding claims, wherein B is -Y-Z-.

9. A compound according to any one of the preceding claims, wherein Y is a bond, -O-, -NH-, -S(O)₂- or-C(O)-.

10. A compound according to any one of the preceding claims, wherein Z is - C(R⁶⁰R⁶¹)- or -C(R⁶⁰R⁶¹)-C(R⁶²R⁶³)-, wherein
R⁶⁰, R⁶¹, R⁶², R⁶³ are independently hydrogen, -C(O)NH₂, -COOH, -CH₂-COOH,
-CH₂-C(O)NH₂, fluoro, methyl, cyclopropyl or
R⁶⁰ and R⁶¹ form a cyclopropyl ring or
R⁶² and R⁶³ form a cyclopropyl ring or
R⁶⁰ and R⁶² form a cyclopropyl or cyclobutyl ring.

11. A compound according to claim 11, wherein R⁶⁰, R⁶¹, R⁶², R⁶³ are independently hydrogen, fluoro or -C(O)NH₂.

12. A compound according to any of the preceding claims, wherein G is -CH(R⁶⁴)-C(R⁶⁵R⁶⁶)-; wherein R⁶⁴ is hydrogen, F, methyl, -CH₂F, -CHF₂, CF₃ or cyclopropyl, R⁶⁵, R⁶⁶ are independently hydrogen, , methyl, -CH₂F, -CHF₂, CF₃ or cyclopropyl or R⁶⁵, R⁶⁶ form together cyclopropyl.

13. A compound according to any one of the preceding claims, wherein G is -CH₂-CH₂-.

14. A compound according to any one of the preceding claims, wherein D is -CH₂-, -CF₂-, -CH(CH₃)-, -C(CH₃)₂- or D¹-D², where D¹ and D² are independently -CH₂-, -CF₂-, -CH(CH₃)- or -C(CH₃)₂- and wherein D² is optionally -CH₂-NH-.

15. A compound according to claim 15, wherein D is -CH₂-, -CH(CH₃)-, -CH₂-CH₂-, -CH₂-CF₂ or -CH₂-CH₂-NH-.

16. A compound according to any one of the preceding claims, wherein -E is selected from the group consisting of phenyl; heterocycle containing 1, 2 or 3 heteroatoms, which are the same or different and selected from the group consisting of -O-, -N=, -N(O)- and -NH-; and heterobicycle containing 1, 2 or 3 heteroatoms, which are the same or different and selected from the group consisting of -O-, -N=, and -NH-; and wherein E is optionally substituted with 1 or 2 substituents which are the same or different and selected from the group consisting of CN, F, Cl, C₁₋₄ alkyl, OH, O-C₁₋₄ alkyl, NH₂, NH-C₁₋₄ alkyl, N(C₁₋₄ alkyl)₂, C(O)NH₂, C(O)NH-C₁₋₄ alkyl, and C(O)N(C₁₋₄ alkyl)₂, wherein each C₁₋₄ alkyl is optionally substituted with one or more substituents independently selected from OH and F.

17. A compound according to claim 17, wherein -E is phenyl, pyridine, benzimidazole, indazole, quinoline, isoquinoline, pyridine-(N)-oxide, benzothiophene, indole, azaindole, benzofuran, benzisoxazole, benzoxazole, benzothiazole.

18. A compound according to any one of the preceding claims, wherein -E is selected from the group consisting of 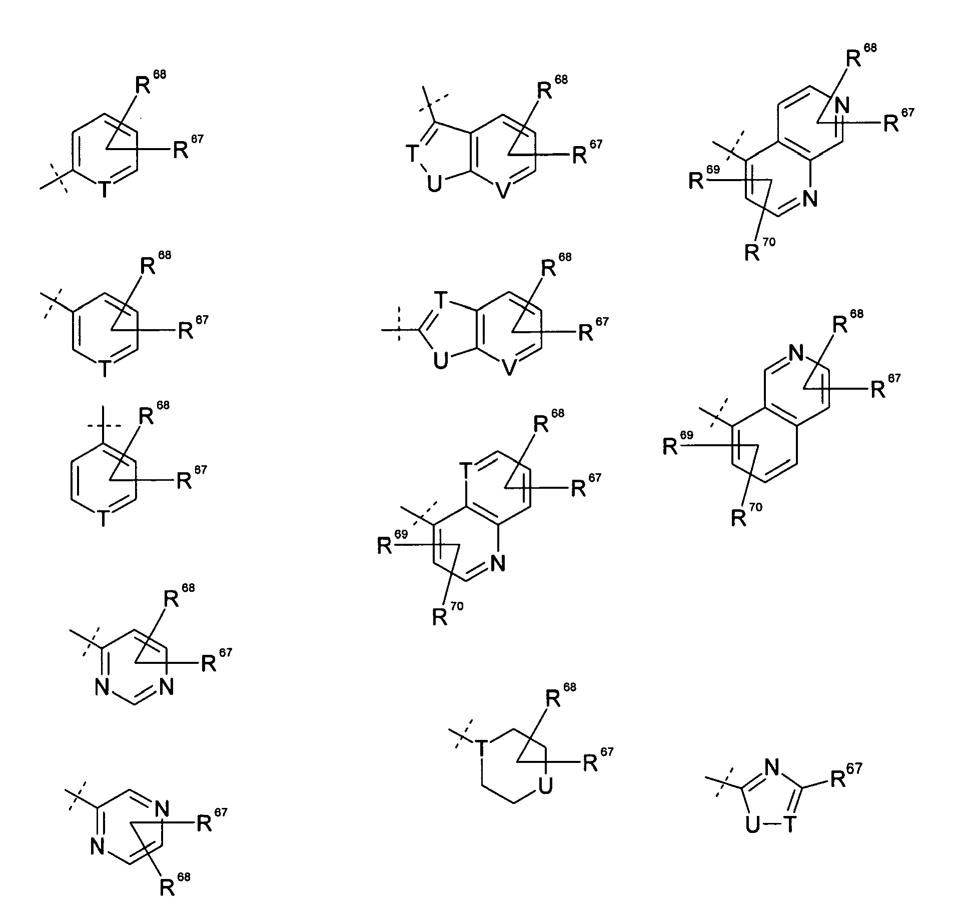 wherein
T and V are independently =CH-, =CR⁷¹-, =N- or =N(O)-;
U is -NH-, -NR⁷²-, -O-, or -S-, wherein
R⁶⁷, R⁶⁸, R⁶⁹, R⁷⁰, R⁷¹ are independently selected from the group consisting of
hydrogen;
C₃₋₆ cycloalkyl;
E⁶;
E⁷;
halogen;
CN;
CNO;
-N(R⁷³R⁷⁴);
-OH; and
-COOR⁷⁵ or -C(O)NR⁷⁶R⁷⁷;
and wherein R⁷², R⁷³, R⁷⁴ are independently
hydrogen;
C₁₋₄ alkyl; or
-C(O)-C₁₋₄ alkyl;
and wherein R⁷⁵, R⁷⁶, R⁷⁷ are independently
hydrogen; or
C₁₋₄ alkyl;
E⁶ is selected from the group consisting of C₁₋₆ alkyl; -O-C₁₋₆ alkyl; and -N(R⁷⁸)-C₁₋₆ alkyl, wherein the C₁₋₆ alkyl group is optionally substituted with one or more of
F;
CN;
CNO;
-N(R⁷⁹R⁸⁰);
phenyl, optionally substituted with chloro;
heterocycle containing 1, 2, 3 or 4 heteroatoms, which are the same or different and selected from the group consisting of -O-, -S-, -S(O)-, -S(O₂)-, -N=, -N(O)= and -N(R⁸¹)-, optionally substituted with chloro;
and/or E⁶ is optionally interrupted by one or more of oxygen;
and wherein R⁷⁸, R⁷⁹, R⁸⁰, R⁸¹ are independently hydrogen, C₁₋₄alkyl;
E⁷ is selected from the group consisting of E⁸; -O-E⁸; -N(R⁸²)-E⁸; and -C(O)-E⁸, wherein
E⁸ is phenyl or heterocycle containing 1, 2, 3 or 4 heteroatoms, which are the same or different and selected from the group consisting of -O-, -S-, -S(O)-, -S(O₂)-, -N=,
-N(O)= and -N(R⁸³)-; and wherein E⁸ is optionally substituted with chloro or -N(R⁸⁴ R⁸⁵);
and wherein R⁸², R⁸³, R⁸⁴, R⁸⁵ are independently hydrogen or C₁₋₄ alkyl.

19. A compound according to claim 19, wherein R⁶⁷, R⁶⁸, R⁶⁹, R⁷⁰, R⁷¹ are independently selected from the group consisting of hydrogen, fluoro, chloro, cyano, phenyl, chlorophenyl, methyl, methoxy, amino, monomethyl amino, dimethyl amino, pyrrolyl, diazolyl, triazolyl, and tetrazolyl.

20. A compound according to claim 1 selected from the group consisting of:

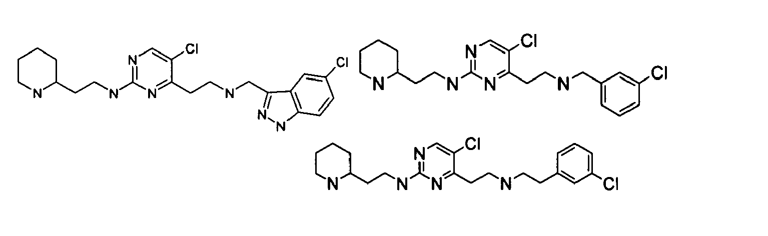

21. A prodrug of a compound according to any one of the claims 1 to 21.

22. A pharmaceutical composition comprising a compound or a mixture of compounds or a pharmaceutically acceptable salt thereof according to any one of the claims 1 to 21 together with a pharmaceutically acceptable carrier.

23. A pharmaceutical composition comprising a prodrug according to claim 22 or a mixture of prodrugs or prodrugs and compounds according to any one of the claims 1 to 21 or a pharmaceutically acceptable salt together with a pharmaceutically acceptable carrier.

24. A pharmaceutical composition according to claim 23 or 24, additionally comprising one or more known anticoagulants.

25. A compound or a pharmaceutically acceptable salt of any one of the claims 1 to 21 for use as a medicament.

26. A prodrug or a pharmaceutically acceptable salt of claim 22 for use as a medicament.

27. Use of a compound or a pharmaceutically acceptable salt of any of the claims 1 to 21 for the manufacture of a medicament for the treatment or prophylaxis of thromboembolism, thrombosis, artherosclerosis, unstable angina, refractory angina, myocardial infarction, transient ischemic attacks, atrial fibrillation, thrombotic stroke, embolic stroke, deep vein thrombosis, disseminated intravascular coagulation, ocular build up of fibrin, and reocclusion or restenosis of recanalized vessels.

28. Use of a prodrug or a pharmaceutically acceptable salt of claim 22 for the manufacture of a medicament for the treatment or prophylaxis of thromboembolism, thrombosis, artherosclerosis, unstable angina, refractory angina, myocardial infarction, transient ischemic attacks, atrial fibrillation, thrombotic stroke, embolic stroke, deep vein thrombosis, disseminated intravascular coagulation, ocular build up of fibrin, and reocclusion or restenosis of recanalized vessels.

29. Use of a compound according to any one of the claims 1 to 21 or a prodrug according to claim 22 as an anticoagulant or thrombin inhibitor.
